# EUROPEAN PATENT APPLICATION

(11) **EP 4 523 721 A1**
(43) Date of publication of application: **19.03.2025**
(21) Application number: 24199947.3
(22) Date of filing: 12.09.2024
(51) Int. Cl.: A61M 5/142, A61B 5/00, A61B 5/145, A61M 5/172

(54) **ADHESIVE PATCH AND SYSTEM FOR WEARABLE MEDICAL DEVICE**

(30) Priority: 12.09.2023 US 202363582043 P
(71) Applicant: Insulet Corporation, Acton, MA 01720 (US)
(72) Inventor: BUSSIERE, John, Littleton, MA 01460 (US); LEE, Joon Bok, Acton, MA 01720 (US); ZHENG, Yibin, Hartland, WI 53029 (US); ZADE, Ashutosh, San Diego, CA 92128 (US); O'CONNOR, Jason, Acton, MA 01720 (US)
(74) Representative: Peterreins Schley

(57) **Abstract**

In an aspect, an adhesive patch is presented. The adhesive patch has a top surface configured to adhere to a wearable medical device. The adhesive patch has a bottom surface opposite the top surface, wherein the bottom surface includes an adhesive layer configured to adhere to a user's skin. The adhesive patch has a first perforation extending through the top and bottom surfaces, the first perforation configured to receive a piercing element from the wearable medical device.

## Description

### CROSS-REFERENCE TO RELATED APPLICATIONS

The application claims priority to, and the benefit of, U.S. Provisional App. No. 63/582,043, filed September 12, 2023, the entirety of which is incorporated herein by reference.

### FIELD

The present subject matter generally relates to adhesive patches. In particular, the present subject matter relates to adhesive patches and systems for wearable medical devices.

### BACKGROUND

The high-volume production and distribution needs of a body worn device with an integrated adhesive pad for function of a secured attachment to device users body does not easily allow for a variety of "adhesive pad" options to meet the extreme variable needs of individual uniqueness and physiology of their skin.

### SUMMARY OF THE DISCLOSURE

In an aspect, an adhesive patch is presented. The adhesive patch has a top surface configured to adhere to a wearable medical device. The adhesive patch has a bottom surface opposite the top surface, wherein the bottom surface includes an adhesive layer configured to adhere to a user's skin. The adhesive patch has a first perforation extending through the top and bottom surfaces, the first perforation configured to receive a piercing element from the wearable medical device.

In another aspect, a system for a wearable medical device is presented. The system includes a wearable medical device configured to deliver a medication to a user. The system includes an adhesive patch coupled to the wearable medical device. The adhesive patch includes a top surface, wherein the top surface is configured to adhere to the wearable medical device. The adhesive patch includes a bottom surface opposite the top surface, wherein the bottom surface includes an adhesive layer configured to adhere to a user's skin. The adhesive patch includes a first perforation extending through the top and bottom surfaces, the first perforation configured to receive a piercing element from the wearable medical device.

These and other aspects and features of non-limiting embodiments of the present invention will become apparent to those skilled in the art upon review of the following description of specific non-limiting embodiments of the invention in conjunction with the accompanying drawings

### BRIEF DESCRIPTION OF THE DRAWINGS

FIG. 1 is an exemplary embodiment of an adhesive patch;
FIGS. 2A-B illustrate an adhesive patch with a wearable medical device;
FIGS. 3A-B illustrates another embodiment of an adhesive patch with a wearable medical device;
FIG. 4 illustrates a side view of an adhesive patch with a wearable medical device;
FIG. 5 illustrates an exemplary embodiment of a plurality of adhesive patches with skin tone coloring;
FIG. 6 illustrates an exemplary embodiment of a plurality of adhesive patches with various visual aesthetics;
FIGS. 7A-C illustrate exemplary embodiments of adhesive patches with various perforation patterns;
FIG. 8 illustrates an exemplary embodiment of an adhesive patch with a shear force applied;
FIG. 9 illustrates an exemplary embodiment of a dual adhesive patch;
FIG. 10 is a graph illustrating bond strengths of adhesives of a dual adhesive patch;
FIG. 11 illustrates an exemplary embodiment of system for medicament filling;
FIG. 12 illustrates another exemplary embodiment of a system for medicament filling;
FIG. 13 illustrates another exemplary embodiment of a system for medicament filling;
FIG. 14 illustrates yet another exemplary embodiment of a system for medicament filling;
FIG. 15 illustrates an exemplary embodiment of a dual gradient syringe; and
FIG. 16 illustrates an exemplary embodiment of a wearable medical device.

### DETAILED DESCRIPTION

In the following description, for the purposes of explanation, numerous specific details are set forth in order to provide a thorough understanding of the present invention. It will be apparent, however, that the present invention may be practiced without these specific details. As used herein, the word "exemplary" or "illustrative" means "serving as an example, instance, or illustration." Any implementation described herein as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims.

At a high level, aspects of the present disclosure relate to adhesive patches. Aspects of the present disclosure can be used to provide easily removeable adhesive patches. In some embodiments, aspects of the present disclosure can be used to adhere a wearable medical device to a user. Aspects of the present disclosure can be used to provide a pathway for a cannula or other insertion mechanism to a user's skin. In some embodiments, aspects of the present disclosure can provide a color coded schema matching functions of wearable medical devices to colors of an adhesive pad, which may allow for easier implementation of wearable medical devices.

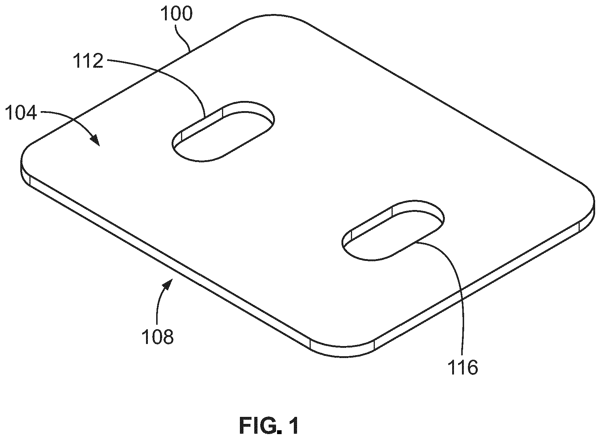

Referring now to FIG. 1, an adhesive patch 100 is shown. The adhesive patch 100 may be made from a cloth, polymer, or other suitable material. For instance, in some embodiments, the adhesive patch 100 may be made from a non-woven single coated polyester tape with polyacrylic adhesive and a siliconized release paper. In some embodiments, the adhesive patch 100 may be made from one or more biocompatible materials. The adhesive patch 100 may be made from one or more polymeric combinations of adhesives and tape that may be non-toxic to human skin. The adhesive patch 100 may be flexible, allowing the adhesive patch 100 to curve, bend, or otherwise distort its shape. In some embodiments, the adhesive patch 100 may be shaped, such as, without limitation, as a square, rectangle, and the like. The adhesive patch 100 may, in an embodiment, be shaped as a rectangle with rounded corners. In some embodiments, the adhesive patch 100 may be about 3 inches long and 2 inches tall, without limitation. In some embodiments the adhesive patch 100 may be between about 2 cm to about 15 cm long and 1.5 to about 10 cm tall. In other embodiments, the adhesive patch 100 may be greater or less than 3 inches long and greater or less than 2 inches tall. The adhesive patch 100 may have a thickness of about 0.25 inches. In other embodiments, the adhesive patch 100 may have a thickness of greater than or less than 0.25 inches. In some embodiments, the adhesive patch may have a thickness between 0.5 mm to about 5mm.

In some embodiments, the adhesive patch 100 may have one or more layers that may have a thickness of, but not limited to, about 0.008 inches to about 0.016 inches. A length and/or width of the adhesive patch 100 may have a perimeter exceeding an outer most perimeter of a wearable medical device. In some embodiments, the adhesive patch 100 may have a length and/or width greater than a length and/or width of a wearable medical device by about 0.1 in to about 0.5 inch, without limitation.

The adhesive patch 100 may include top surface 104 and/or bottom surface 108. The top surface 104 may be a surface facing towards a positive y axis value in a Cartesian coordinate system. The bottom surface 108 may be facing opposite the top surface 104 (away from the top surface). For instance, the bottom surface 108 may be facing towards a negative y axis value in a Cartesian coordinate system. In some embodiments, the top surface 104 and the bottom surface 108 may join together at one or more edges of each surface. As a non-limiting example, the top surface 104 may be connected to the bottom surface 108 at each end of a rectangular shape of the top surface 104. In some embodiments, the top surface 104 of the adhesive patch 100 and the bottom surface 108 may combine to form the adhesive patch 100. For instance and without limitation, the top surface 104 and the bottom surface 108 may be shaped the same and/or have the same dimensions.

The top surface 104 may include an adhesive layer. An "adhesive layer" as used in this disclosure is a surface having adhesive properties. An adhesive layer of the top surface 104 may coat an entirety of the top surface 104. In other embodiments, an adhesive layer of the top surface 104 may partially coat the top surface 104. For instance and without limitation, an adhesive layer of the top surface 104 may coat a middle portion and each corner of a rectangle of the top surface 104. An adhesive layer of the top surface 104 may be made of any suitable composition, such as, but not limited to, acrylate-based polymers. An adhesive layer of the top surface 104 may be a pressure sensitive, contact, or other adhesive, without limitation. In some embodiments, an adhesive layer of the top surface 104 may be about 1 mm thick. In other embodiments, an adhesive layer of the top surface 104 may be greater than or less than 1 mm thick. In some embodiments, the adhesive layer may be about 0.05 mm to about 2mm thick. In some embodiments, the bottom surface 108 may have an adhesive layer. An adhesive layer of the bottom surface 108 may be the same as that of an adhesive layer of the top surface 104. In other embodiments, an adhesive layer of the bottom surface 108 may have different adhesive properties than that of an adhesive layer of the top surface 104. Adhesive properties may include, but are not limited to, strength, adhesive duration, volume, thickness, heat resistance, and the like. In particular, the adhesive property may relate to the adhesive strength. For instance, and without limitation, an adhesive layer of the bottom surface 108 may have a higher adhesive strength and/or a higher adhesive volume than an adhesive layer of the top surface 104. The adhesive strength may be determined according to ASTM D903-98(2017).

An adhesive layer of the top surface 104 may have adhesive properties specific to a wearable medical device, which may be placed on top of the adhesive layer. For instance, an adhesive layer of the top surface 104 may have a thinner layer of adhesive, a stronger adhesive strength, and the like, than that of an adhesive layer of the bottom surface 108. In some embodiments, an adhesive layer of the bottom surface 108 may have adhesive properties for specific body parts of a user, such as, but not limited to, arms, legs, stomachs, backs, buttocks, and the like. As a non-limiting example, an adhesive layer of the bottom surface 108 may be thicker to prevent the adhesive patch 100 from falling of a user's arm, which may be prone to bumping into objects and/or people. In some embodiments, the bottom surface 108 may have an adhesive layer while the top surface 104 may have a non-adhesive layer.

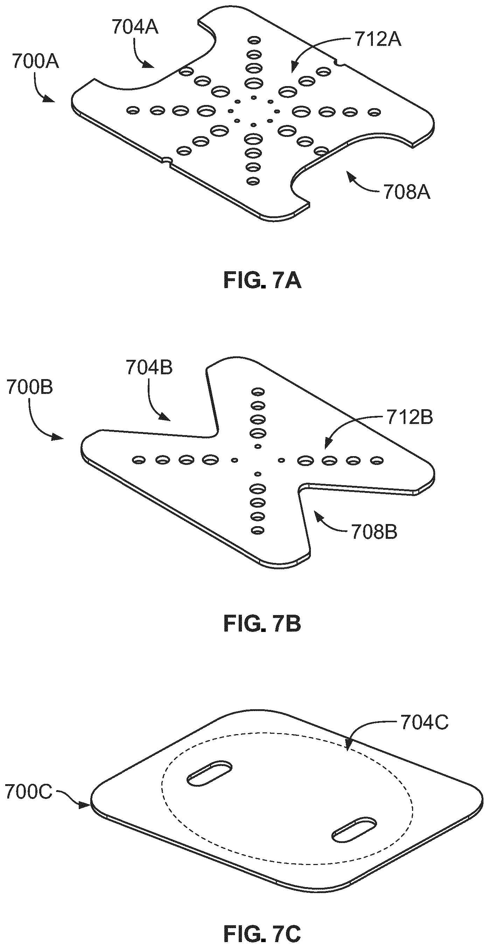

Still referring to FIG. 1, in some embodiments, the adhesive patch 100 may include first perforation 112. A "perforation" as used in this disclosure is a hole passing through an object. The first perforation 112 may be shaped as, without limitation, a circle, rectangle, square, oval, and the like. The first perforation and or second perforation may have a (maximum) diameter of between about 0.5 mm to about 20 mm, more specifically between about 1 mm to about 10 mm and in particular between about 2 mm to about 5 mm. The first perforation and or second perforation may have a (maximum) edge length of between about 0.5 mm to about 20 mm, more specifically between about 1 mm to about 10 mm and in particular between about 2 mm to about 5 mm. The first perforation 112 may be positioned at an end of the adhesive patch 100, such as, without limitation, a left, right, top, or bottom end. In some embodiments, the first perforation 112 may be positioned about 0.5 in (1.27 cm) to about 1.25 in (3.175 cm) from a leftmost side of the adhesive patch 100, without limitation. In other embodiments, the first perforation 112 may be positioned greater than or less than about 2 mm from a leftmost side of the adhesive patch 100. The adhesive patch may include a second perforation 116. The second perforation 116 may be the same as that of the first perforation 112. In other embodiments, the second perforation 116 may differ from the first perforation 112, such as by shape, size, position, and the like, without limitation. The second perforation 116 may be positioned about 2 mm from a rightmost side of the adhesive patch 100. In some embodiments, the first perforation 112 may be positioned about 4 mm away from the second perforation 116. The first perforation 112 and the second perforation 116 may extend through the top surface 104 and the bottom surface 108, allowing for one or more objects to pass through the adhesive patch 100. The first perforation 112 may be positioned about 4 mm to about 6 mm away from the second perforation 116, without limitation. In other embodiments, the first perforation 112 may be positioned less than 4 mm or greater than 6 mm away from the second perforation 116. In some embodiments, the adhesive patch 100 may have three or more perforations, as described in further detail below with reference to FIG. 7.

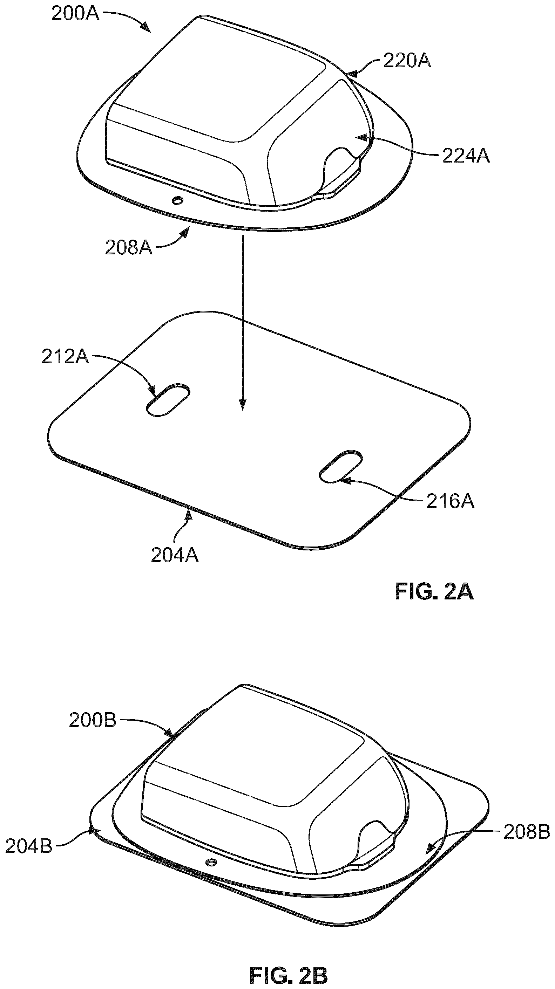

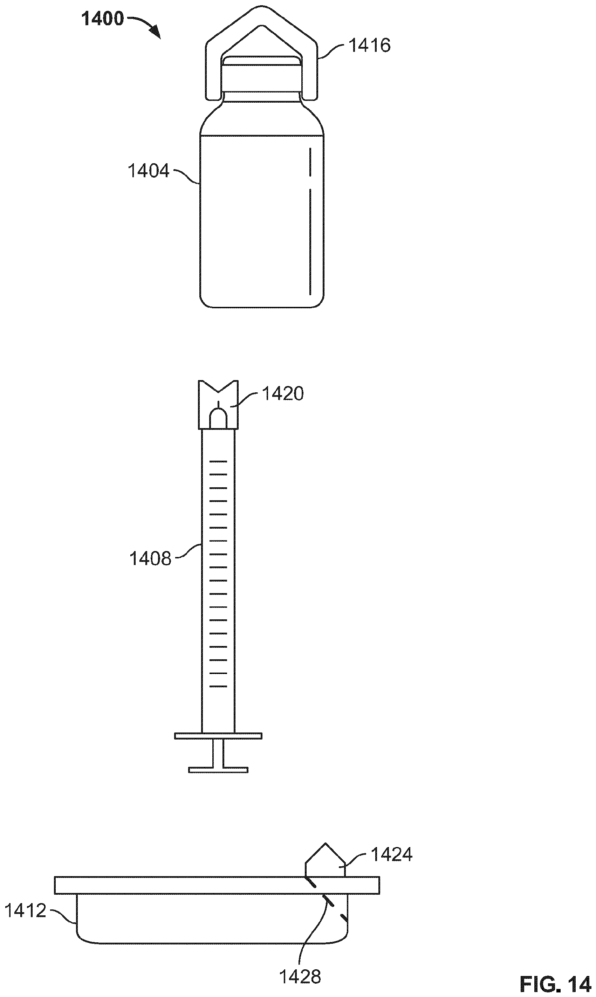

Referring now to FIG. 2A, a wearable medical device 200A positioned next to adhesive patch 204A is shown. The wearable medical device 200A may include, without limitation, an insulin pump. In some embodiments, the wearable medical device 200A may include a sensing device and/or a cannula. The wearable medical device 200A may be described further below with reference to FIG. 14.

The adhesive patch 204A may be the same as the adhesive patch 100 as described above with refence to FIG. 1. The adhesive patch 204A may include first perforation 212A and/or second perforation 214A. The first perforation 212A and the second perforation 214A may be as described above with reference to first perforation 120 and second perforation 124 in FIG. 1.

The wearable medical device 200A may, in some embodiments, have an adhesive surface 208A. The adhesive surface 208A may be shaped such as, but not limited to, in a circular, square, ovular, and/or other shape. The adhesive surface 208A may have one or more adhesive layers. For instance, the adhesive surface 208A may have a first adhesive layer on a bottom of the adhesive surface 208A and a second adhesive layer on a top of the adhesive surface 208A. The adhesive surface 208A may be coupled to the wearable medical device 200A. In some embodiments, the adhesive layer 208A may be part of the wearable medical device 200A. For instance, the wearable medical device 208A may be manufactured with the adhesive surface 208A. In some embodiments, the adhesive surface 208A may be part of an outer layer of the wearable medical device 200A. The wearable medical device 200A may have an outer layer and an inner layer, each of which may be composed of rubber, plastic, and the like. The outer layer and the inner layer may form a shell or housing 220A for the wearable medical device 200A. The wearable medical device 200A may be positioned within the housing 220A, which may be made of an outer and inner layer. The housing 220A may be shaped, without limitation, as a rectangle, oval, square, circle, and/or other shapes or r any combination thereof. The housing 220A may include port 224A. The port 224A may allow access to the wearable medical device 200A through the housing 220A. In some embodiments, the port 224A may be circular, rectangular, and/or other shapes.

Still referring to FIG. 2A, the wearable medical device 200A may be applied to the adhesive patch 204A. The adhesive layer 208A may be affixed or otherwise coupled to a top surface of the adhesive patch 204A. An adhesive layer of the adhesive patch 204A and the adhesive layer 208A may interact, forming a adhesive bond between the adhesive patch 204A and the wearable medical device 200A. The wearable medical device 200A may include one or more piercing elements, such as, but not limited to, cannulas, sensing devices, and the like. The wearable medical device 200A may be positioned on top of the adhesive patch 204A such that a first piercing element of the wearable medical device 200A may align with the first perforation 212A of the adhesive patch 204A and/or a second piercing element of the wearable medical device 200A may be aligned with the second perforation 216A of the adhesive patch 204A. The first perforation 212A and the second perforation 216A may allow for one or more piercing elements of the wearable medical device 200A to pass through the adhesive patch 204A and into a body part of a user.

Referring now to FIG. 2B, an illustration of wearable medical device 200B adhered to adhesive patch 204B is shown. The wearable medical device 200B may be the same as that of the wearable medical device 200A, the adhesive layer 208B, and the adhesive patch 204B may be the same as that of the adhesive patch 204A as described above with reference to FIG. 2A.

The wearable medical device 200B may be placed or otherwise positioned onto the adhesive patch 204B through manual force, automated manufacturing tools, and the like. In some embodiments, an adhesive of an adhesive layer of the adhesive patch 204B may be the same as an adhesive of the adhesive layer 208B. In other embodiments, an adhesive of the adhesive layer 208B and an adhesive of an adhesive layer of the adhesive patch 204B may differ. In some embodiments, the adhesive patch 204B coupled to the wearable medical device 200B may be placed on a body part of a user, such as, but not limited to, an arm, leg, stomach, back, and the like.

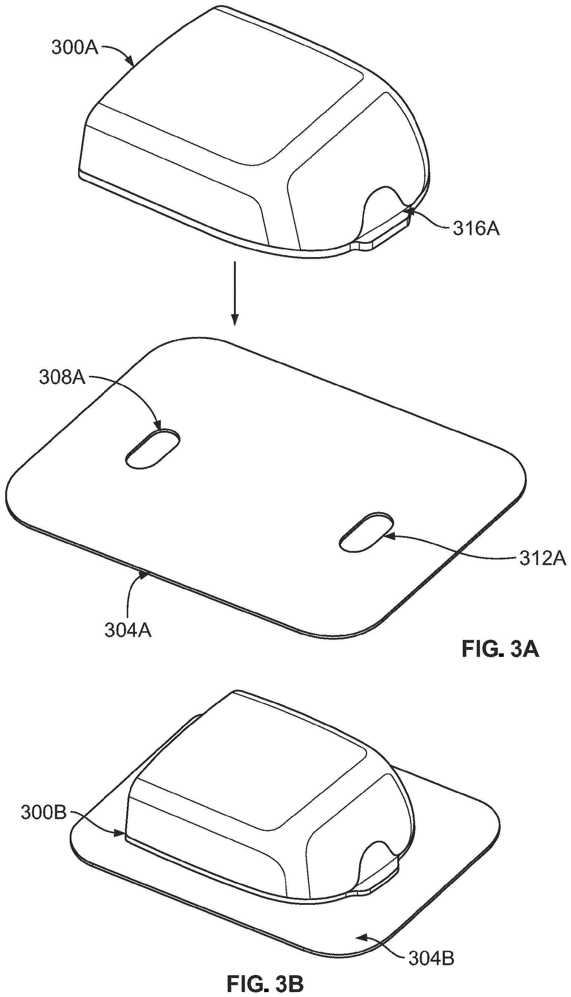

Referring now to FIG. 3A, another embodiment of a wearable medical device 300A and an adhesive patch 304A is presented. The wearable medical device 300A and/or the adhesive patch 304A may be the same as that of the wearable medical device 200A and the adhesive patch 204A as described above, without limitation. In some embodiments, the wearable medical device 300A may lack an adhesive layer. A bottom surface of the wearable medical device 300A may be part of a housing 316A, which may be made of a polymer, plastic, and/or other material, without limitation. The adhesive patch 304A may include first perforation 308A and/or second perforation 312A, which may be described above with reference to FIG. 2A without limitation. One or more piercing elements of the wearable medical device 300A may align with a positioning of the first perforation 308A and/or the second perforation 312A. For instance, and without limitation, a cannula of the wearable medical device 300A may align with the first perforation 308A and a sensing device of the wearable medical device 300A may align with the second perforation 312A. An adhesive layer of the adhesive patch 304A may be configured to affix the wearable medical device 300A to a top surface of the adhesive patch 304A.

Referring now to FIG. 3B, an embodiment of a wearable medical device 300B adhered to an adhesive patch 304B is shown. The wearable medical device 300B and the adhesive patch 304B may be the same as the wearable medical device 200A and the adhesive patch 204A as described above with reference to FIG. 2A, without limitation. The wearable medical device 300B may be adhered to the adhesive patch 304B through manual force, automatic manufacturing tools, and the like. The adhesive patch 304B coupled to the wearable medical device 300B may be placed on a body part of a user, such as, but not limited to, a leg, arm, back, stomach, and the like.

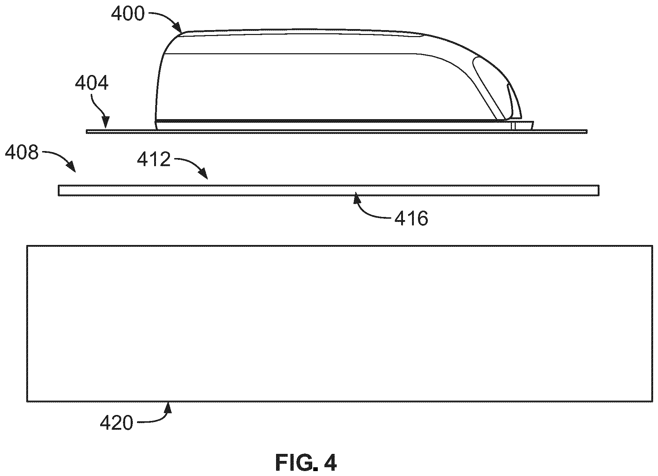

Referring now to FIG. 4, a side profile view of a wearable medical device 400 and an adhesive patch 404 is presented. The wearable medical device 400 and the adhesive patch 404 may be as described above with reference to FIGS. 2A-B.

The wearable medical device 400 may include adhesive layer 404. The adhesive layer 404 may be part of a housing of the wearable medical device 400. In some embodiments, the adhesive layer 404 may be positioned at a bottom surface of the wearable medical device 400. The adhesive layer 404 may include any suitable adhesive, such as, but not limited to, polymers. Polymers may include non-toxic, biocompatible, and/or other polymers. The adhesive layer 412 may be configured to adhere to the top surface 412 of the adhesive patch 408. In some embodiments, the top surface 412 has an adhesive layer. In other embodiments, the top surface 412 may include a non-adhesive layer, such as a plastic, cloth, or other material, without limitation. The adhesive layer 404 of the wearable medical device 400 may adhere to or otherwise couple to the top surface 412 of the adhesive patch 408. The adhesive patch 408 may include a bottom surface 416. The adhesive patch 408, top surface 412, and bottom surface 416 may be as described above with reference to FIG. 1.

The bottom surface 416 may include an adhesive layer. In some embodiments, an adhesive layer of the bottom surface 416 may be the same as that of an adhesive of the adhesive layer 404 of the wearable medical device 400. In other embodiments, an adhesive layer of the bottom surface 416 may be different from an adhesive of the adhesive layer 404 of the wearable medical device 400. An adhesive layer of the bottom surface 416 may be selected and/or applied to the adhesive patch 408 for a plurality of factors, such as, but not limited to, body parts the adhesive patch 408 may be applied to, type of wearable medical device 400, and the like. In some embodiments, both the top surface 412 and the bottom surface 416 of the adhesive patch 408 may have an adhesive surface. In other embodiments, the top surface 412 may have a non-adhesive surface and the bottom surface 416 may have an adhesive surface, or vice versa. The bottom surface 416 may be configured to adhere or otherwise couple to a user's skin 420. The user's skin 420 may include skin of, but not limited to, arms, legs, backs, stomachs, and the like. The bottom surface 416 may have an adhesive layer specific to a body part the users skin 420 may be located on. For instance, the user's skin 420 may be located on an arm of the user which may be prone to bumping onto objects. In some embodiments, the bottom surface 416 may have a stronger adhesive to account for potential bumping of the wearable medical device 400 into one or more objects when the adhesive patch 408 is placed on an arm of the user. In some embodiments, the bottom surface 416 may have an adhesive to account for a skin sensitivity of the user's skin 420. For instance, a latex-free adhesive may be used in an adhesive layer of the bottom surface 416.

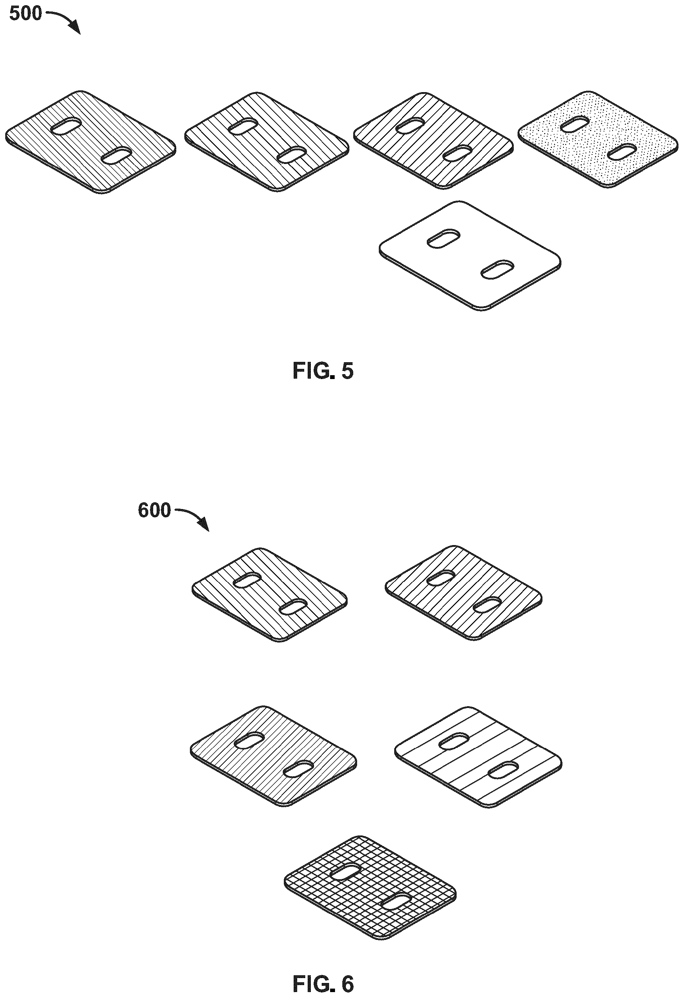

Referring now to FIG. 5, a plurality of adhesive patches 500 with natural skin colors is shown. Each adhesive patch of the plurality of adhesive patches 500 may be colored to match lighter skin tones, medium skin tones, and/or darker skin tones of a user. Each adhesive patch of the plurality of adhesive patches 500 may be dyed with colorants, colored with a silk screen, pad printed, laser printed, and/or other colored by other coloring processes. Each adhesive patch of the plurality of adhesive patches 500 may be colored with Pantone, cyan magenta yellow and key (CMYK), red green blue (RGB), and/or Hex color specifications of one or more colors and/or patterns. In some embodiments, an adhesive patch may be transparent. A transparent adhesive patch may allow for a natural skin tone of a user to be seen through the transparent adhesive patch. In some embodiments, a top surface of an adhesive patch may be colored while a bottom surface of the adhesive patch may not be colored to match a natural skin color. In other embodiments, both a top surface and a bottom surface of an adhesive patch made be colored to match a natural skin color.

Referring now to FIG. 6, a plurality of adhesive patches 600 with varying aesthetics is presented. An adhesive patch of the plurality of adhesive patches 600 may be colored, without limitation, blue, red, green, yellow, and/or any combination thereof. In some embodiments, an adhesive patch of the plurality of adhesive patches 600 may have a pattern, such as, but not limited to, stripes, stars, swirls, and/or other patterns. In some embodiments, an adhesive patch may have a letter, symbol, or other character that may correspond to one or more body parts of a user. For instance, an "S" may be printed on an adhesive patch and may correspond to a stomach of a user for which the adhesive patch may be placed, without limitation. As a non-limiting example, a star pattern may represent an adhesive patch meant for a toddler, a striped pattern may represent an adhesive patch meant for a teenager, and the like. In some embodiments, a user may select one or more colors, patterns, and/or other visual aspects of an adhesive patch.

An adhesive patch may be color coded according to a color code schema. A "color code schema" as used in this disclosure is a system of colors and/or patterns corresponding to one or more properties and/or functions of a wearable medical device, and/or adhesive patch. For instance, a color of an adhesive patch may be representative of a body part the adhesive patch may adhere to. As a non-limiting example, a blue color of an adhesive patch may correspond to an arm of a user, a green color may correspond to a back of a user, and a red color may correspond to a stomach of a user. In some embodiments, a color of an adhesive patch may correspond to a different adhesive strength of the adhesive patch. As a non-limiting example, a yellow color may correspond to a medium adhesive strength, a purple color may correspond to a light adhesive strength, and an orange color may correspond to a strongest adhesive strength. In some embodiments, a color of an adhesive patch may correspond to a type of wearable medical device. As a non-limiting example, a blue color may correspond to an insulin pump, a yellow color may correspond to an analyte sensor, such as a blood glucose sensor, and a red color may correspond to a heart rate monitor. In some embodiments, a color of an adhesive patch may correspond to different adhesive types. For instance, and without limitation, a teal color of an adhesive patch may correspond to a latex-free adhesive, and a neon green color may correspond to an adhesive comprising latex. In some embodiments, a color of an adhesive patch may correspond to a basal rate of a medicament, a total daily medicament (TDM) value, a bolus delivery value of a medicament, and the like. For instance, and without limitation, a medicament may include insulin and a wearable medical device may include an insulin pump. As a non-limiting example, a blue color of an adhesive patch may correspond to a basal rate of 2U/hr of a insulin and a purple color may correspond to a basal rate of 3.5U/hr of insulin.

An adhesive patch may have a colorable surface. For instance, a top surface and/or a bottom surface of an adhesive patch may be made of a material that retains ink, lead, paint, and/or other art mediums. For instance and without limitation, a top surface of an adhesive patch may be made from a white plastic material, a neutral colored markable polymer, and the like. In some embodiments, an adhesive patch may be provided with a writing kit. A writing kit may include one or more art tools such as, but not limited to, pens, pencils, brushes, stickers, paint, and the like. A user may apply their own style to an adhesive patch, such as through the one or more art tools described above, without limitation. In some embodiments, an adhesive patch may be colored and/or patterned by an automated manufacturing process.

In some embodiments, an adhesive patch of the plurality of adhesive patches 600 may have a texture, such as, without limitation, soft, rough, bumpy, smooth, and the like. A texture of an adhesive patch may be selected for a variety of factors. For instance, and without limitation, a smooth texture of an adhesive patch may be selected for an adhesive patch that may go on a thigh of a user. A rougher texture may be selected for an adhesive patch that may go on a back of a user. In some embodiments, a texture of an adhesive patch may be selected for a stylistic and/or aesthetic option. As a non-limiting example, a star, diamond, and/or other textured pattern may be used.

Referring now to FIG. 7A, an adhesive patch 700A with a perforation pattern 704A is shown. The adhesive patch 700A may be the same as the adhesive patch described above with reference to FIG. 1. The adhesive patch 700A may be shaped rectangularly, circular, ovular, and/or other shapes. In some embodiments, opposing end of the adhesive patch 700A may be shaped to accommodate one or more piercing elements of a wearable medical device. For instance, and without limitation, a top end and/or a bottom end of the adhesive patch 700A may be shaped as a rectangle, square, triangle, and the like. One or more ends of the adhesive patch 700A may have cutout shapes, such as a half rectangle, a half rectangle with rounded corners, a half diamond, a semi-circle, and the like, without limitation. For instance, first side 704A and second side 708A are shown as rounded rectangular cutouts.

The adhesive patch 700A may include perforations 712A. In some embodiments, perforations 712A may be patterned. The perforations 712A may be patterned in spirals, grids, radial lines, diagonal lines, and/or other patterns. For instance, and without limitation, perforations 712A may radiate outwards in lines of circles from a center point of the adhesive patch 700A. As a non-limiting example, the perforations 712A may start from a center point of the adhesive patch 700A, where at the center point a circle of small perforations is positioned. Continuing this example, a line of 4 larger circular perforations may extend radially outward from the center point, for instance in a total of 8 lines. Perforations 712A may provide breathability, comfort, and/or removability ease to the adhesive patch 700A.

Referring now to FIG. 7B, another embodiment of an adhesive patch with patterned perforations is shown. The adhesive patch 700B may be the same as the adhesive patch as described above with reference to FIG. 1. The adhesive patch 700B may have first end 704B and/or second end 708B. The first end 704B of the adhesive patch 700B may be shaped to accommodate one or more piercing elements of a wearable medical device. For instance, the first end 704B and/or the second end 708B may be triangular, rectangular, circular, and/or other-wise shaped. In some embodiments, the first end 704B and/or the second end 708B may have cutout shapes, such as, but not limited to, diamond cutouts, triangular cutouts, circular cutouts, rectangular cutouts, and the like. The adhesive patch 700B may have perforations 712B. Perforations 712B may be patterned, such as, but not limited to, swirls, stripes, butterfly, zig-zag, and/or other patterns. Perforations 712B may be "X" shaped, with two diagonal lines crossing a center point of the adhesive patch 700B. Each line of the two diagonal lines may have four or more circles. In some embodiments, each line of the two diagonal lines may have less than four circles.

Referring now to FIG. 7C, another embodiment of an adhesive patch with perforations is presented. The adhesive patch 700C may be the same as the adhesive patch as described above with reference to FIG. 1. The adhesive patch 700C may include perforations 704C. The perforations 704C may include a set of two perforations, each perforation of the set of two perforations positioned at opposite ends of the adhesive patch 700C. Outline 708C shows a potential placement of a wearable medical device. The adhesive patch 700C may be shaped in any suitable manner to include the outline 708C, such as the shapes of the adhesive patch 700A and/or 700B as described above.

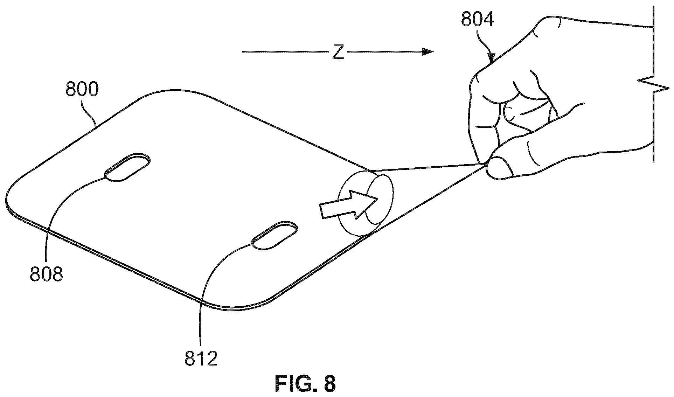

Referring now to FIG. 8, an exemplary embodiment of an adhesive patch with a shear force applied is shown. The adhesive patch 800 may be the same as that of the adhesive patch described above with reference to FIG. 1. The adhesive patch 800 may include first perforation 804 and/or second perforation 808, both of which may be as described above with reference to FIG. 1. A user 812 may apply a shear force 816 to the adhesive pad 800, such as in direction "Z". An adhesive of the adhesive pad 800 may be configured to remove easily upon an appliance of a shear force, while retaining strong adhesive properties against a lifting force. In some embodiments, the adhesive patch 800 may be as described in U.S. Patent No. 5,989,708, filed April 8, 1997, and titled "Removeable Adhesive Tape", which is incorporated by reference herein in its entirety.

The adhesive patches described above may be provided in the form of a kit of parts. The kit of parts may comprise a first adhesive patch and a second adhesive patch. The first adhesive patch and the second adhesive patch may each comprise a top surface configured to adhere to a wearable medical device; a bottom surface opposite the top surface, wherein the bottom surface includes an adhesive layer configured to adhere to a user's skin; and a first perforation extending through the top and bottom surfaces, the first perforation configured to receive a piercing element from the wearable medical device. The first adhesive patch and the second adhesive patch may differ by at least one of and adhesive strength, a colour, dimensions or perforation position. Accordingly, the user is supplied with the option of choosing the adhesive that suits them best from the kit.

In some embodiments, the kit of parts may comprise a wearable medical device, more specifically a disposable wearable medical device, and in particular a disposable wearable insulin delivery device. Disposable wearable medical devices, in particular disposable wearable insulin delivery devices may need to be replaced every couple of days. The user may choose to not apply the disposable wearable delivery device to the same body location all the time. The kit of parts may therefore aid the user in providing optimal adhesive patches for different body regions. For example, the adhesive patch may provide a smaller adhesive patch with a higher adhesive strength to be applied to the arm and a bigger adhesive patch with a lower adhesive strength to be applied to the stomach.

In some embodiments, the first adhesive patch has a different adhesive strength and a different color than the second adhesive patch. Adhesive patches may also have the same size but different adhesive strengths. For example, a more active person may opt to choose an adhesive patch with a higher adhesive strength to prevent disconnection from the skin during sports, whereas a less active person may choose an adhesive patch with a lower adhesive strength to allow easier removal.

In some embodiments, the first adhesive patch and the second adhesive patch comprise a release liner attached to the adhesive layer. The release liner may prevent contamination of the adhesive layer, e.g., during storage.

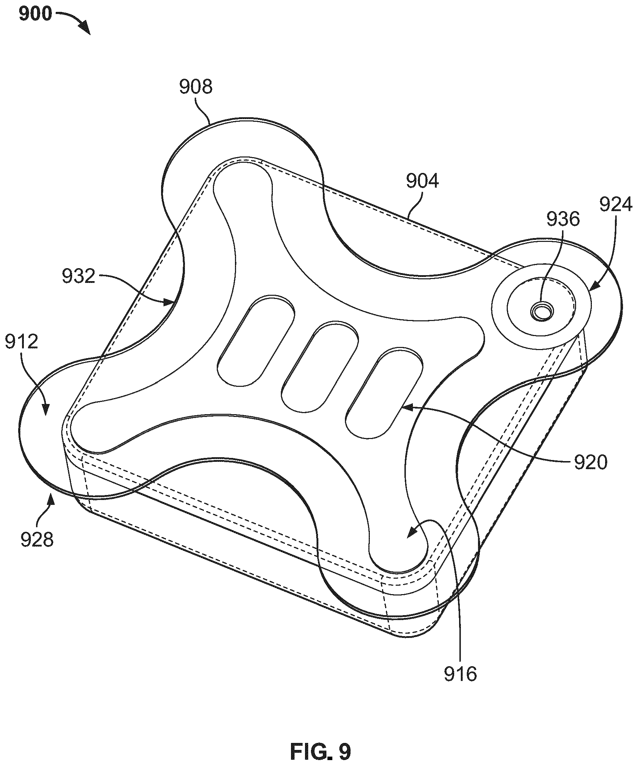

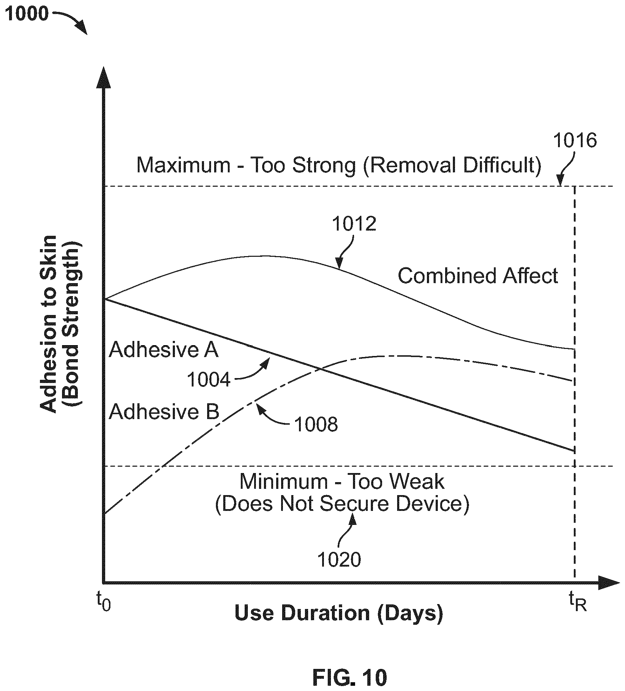

Referring still to FIG. 9, first adhesive 912 may be an adhesive having a stronger immediate bond strength than second adhesive 916. First adhesive 912 may provide an outer perimeter coverage of securement pad 908 while second adhesive 916 may provide an inner area coverage of securement pad 908. First adhesive 912 may be an acrylic-based, rubber-based, or silicone-based adhesive. Likewise, second adhesive 916 may be an acrylic-based, rubber-based, or silicone-based adhesive. Thickness and/or tackiness of first adhesive 912 and/or second adhesive 916 may be adjusted for various skin types. In some embodiments, first adhesive 912 First adhesive 912 may have a greater or lesser thickness than second adhesive 916, and vice versa. First adhesive 912 may have a greater or lesser tack than second adhesive 916, and vice versa. In some embodiments, second adhesive 916 may be thicker than first adhesive 912. First adhesive 912 may have a greater area of coverage and/or contact with a user's skin than second adhesive 916. In some embodiments, securement pad 908 may have a ratio of one or more adhesives to one or more other adhesives. For instance and without limitation, securement pad 908 may be disposed with a ratio of first adhesive 912 to second adhesive 916 of about 1:1, 1.5: 1, 2:1, 2.5: 1, 3:1, 3.5: 1, 4: 1, 4.5: 1, 5:1, or greater than 5:1. Second adhesive 916 may be an adhesive that has an increasing bond strength over time. Bond strengths of first and second adhesive 912 and 916 may be depicted below with reference to FIG. 10.

Referring now to FIG. 10, a graph 1000 illustrating bond strengths of adhesives is shown. Graph 1000 shows multiple lines with a y-axis representing bond strength or adhesion to skin with an x-axis representing time in days. In some embodiments, bond strength may be measured over a span of about 6 to 12 days. Bond strength may be measured in pounds (lbs), newtons (N) or other measurements. In some embodiments, bond strength may be measured as force per area, for instance and without limitation as a pressure per square inch (PSI), newtons per meter squared (Pa), or other values. Line 1016 represents a maximum bond strength that if reached makes removing an adhesive pad difficult. Line 1010 shows a minimum bond strength that if reached makes an adhesive too weak to secure to a wearable medical device and/or user's skin. Graph 1000 shows first adhesive 1004, which may be first adhesive 912 as described above with reference to FIG. 9. As shown, first adhesive 1004 may have a high initial bond strength that may decrease over time. In some embodiments, first adhesive 1004 may have an approximately linear decay of bond strength over time. Graph 1000 shows second adhesive 1008, which may be second adhesive 916 as described above with reference to configurations. FIG. 9. Second adhesive 1008 may have a lower initial bond strength compared to first adhesive 1004. In some embodiments, second adhesive 1008 may have an initial bond strength that may be lower than minimum bond strength 1020. Over time, second adhesive 1008 may increase in a bond strength. In some embodiments, a bond strength of second adhesive 1008 may be equal to a bond strength of first adhesive 1004 at a point in time. Second adhesive 1008 may increase in bond strength and surpass a decreasing bond strength of first adhesive 1004. A combined bond strength 1012 of first adhesive 1004 and second adhesive 1008 may remain approximately linear over time. For instance, combined bond strength 1012 may have an initial bond strength slightly higher than an initial bond strength of first adhesive 1004 and may dip slightly over time compared to the dip in bond strength of first adhesive 1004 over time. A combination of first adhesive 1004 and second adhesive 1008 may allow a relatively stable bond strength of combined bond strength 1012 over time.

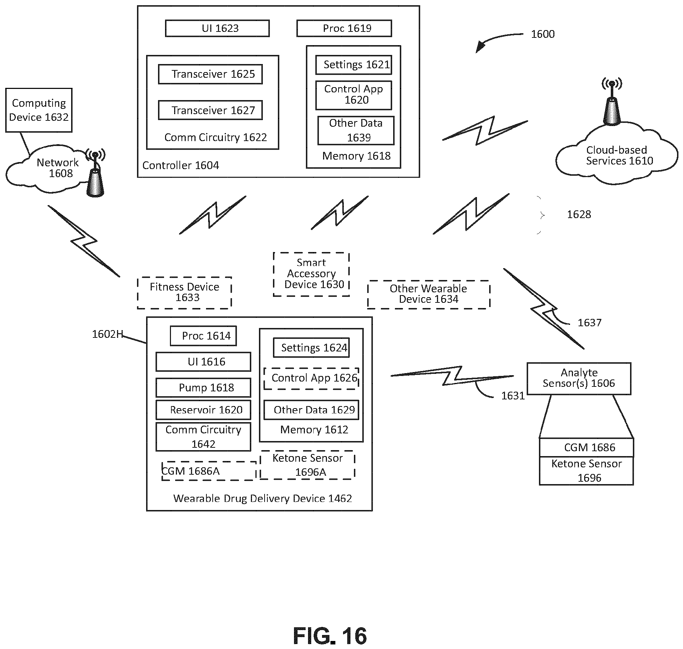

Referring now to FIG. 9, a dual adhesive patch 900 is shown. A "dual adhesive patch" as used in this disclosure refers to an adhesive patch having two or more adhesives. A top side of dual adhesive patch 900 may include an adhesive that may couple the top side of dual adhesive patch 900 to a bottom of wearable medical device 904. Dual adhesive patch 900 may be adhered to and/or coupled to wearable medical device 904. Wearable medical device 904 may be as described below with reference to FIG. 16, without limitation. A bottom of dual adhesive patch 900 may be attachable and/or adherable to a user's skin.

Dual adhesive patch 900 may include securement pad 908. Securement pad 908 may be made from a flexible material, such as a gel, or thin polymeric material. A thin polymeric material may include, but is not limited to, polyurethane, non-woven fabrics, and/or other natural and/or synthetic materials. Securement pad 908 may have a geometry such as, but not limited to, rectangular, square, circular, ovular, diamond, or other geometries. In some embodiments, securement pad 908 may have a radial geometry. A radial geometry may include one or more ends of secured pad 908 extending from a center of securement pad 908. For instance and without limitation, securement pad 908 may extend radially from a center of wearable medical device 904 towards each end of wearable medical device 904. Wearable medical device 904 may be rectangular, circular, square, or other shapes. In embodiments where wearable medical device 904 may be square or rectangular, securement pad 908 may extend radially towards each corner of wearable medical device 904 from a center point of wearable medical device 904. Securement pad 908 may have one or more ends 928. Ends 928 of securement pad 904 may be circular, rectangular, square, or other shapes. In some embodiments, ends 928 of securement pad 908 may be rounded and/or curved.

Securement pad 908 may have four ends 928. Each end 928 may be positioned at a corner or edge of wearable medical device 904, such as in embodiments where wearable medical device 904 may be square or rectangular. Ends 928 may extend past a side of wearable medical device 904. For instance, ends 928 may extend about 0 cm to about 1.5 cm past a side of wearable medical device 904. Ends 928 may extend distally from a corner of wearable medical device 904. Extending distally from a corner of wearable medical device 904 may include extending along a linear path relative to a center of wearable medical device 904 past an end of the corner. For instance, ends 928 may extend diagonally outwards with respect to a center of securement pad 908 and/or wearable medical device 904. In some embodiments, ends 928 of securement pad 908 may be connected by pad side 932. Pad sides 932 may be concave, convex, or linear, without limitation. In some embodiments, pad sides 932 may curve inwards towards a center of wearable medical device 904. In embodiments where pad sides 932 are curved inwards, pad sides 932 may have a highest curvature relative to ends 928 at a median point between two ends 928. A "median point" refers to a middle portion between two or more reference points. A median point of pad sides 932 may align with a median point of a side of wearable medical device 904. In some embodiments, securement pad 908 may have a first set of pad sides 932 and a second side of pad sides 932. A first set of pad sides 932 may be mirrored opposite each other, such as, but not limited to, a left and right side of securement pad 908. A second set of pad sides 932 may be mirrored opposite each other, such as, but not limited to, a top and bottom side of securement pad 908. A second set of pad sides 932 may have a deeper concavity towards a center of securement pad 908 than a fist set of pad sides 932. Securement pad 908 may have a geometric symmetry. For instance and without limitation, securement pad 908 may be horizontally and/or vertically symmetric. A symmetry of securement pad 908 may align with a symmetry of wearable medical device 904, such as in embodiments where wearable medical device 904 may be square or rectangular.

Still referring to FIG. 9, securement pad 908 may be adapted to receive and/or hold one or more adhesives. For instance, securement pad 908 may have first adhesive 912 and second adhesive 916. First adhesive 912 may be an acrylic-based polymer, in some embodiments. Second adhesive 916 may be a silicone-based polymer, in some embodiments. In some embodiments, three or more adhesives may be used. Securement pad 908 may have an outer geometric space and an inner geometric space. An outer geometric space may include ends 928 and/or pad sides 932. An inner geometric space may be spaces of securement pad 908 within areas of ends 928 and/or a center of securement pad 908. First adhesive 912 may be disposed throughout an outer geometry of securement pad 908. For instance, first adhesive 912 may be disposed throughout ends 928 and/or pad sides 932. First adhesive 912 may surround an inner geometric space of securement pad 908 that may be made of second adhesive 916. Second adhesive 916 may be deposited throughout securement pad 908 in an inner geometric space, such as, but not limited to, within a center of securement pad 908 and/or within inner portions of ends 928. In some embodiments, second adhesive 916 may have a mirroring or partially mirroring geometry to that of securement pad 908. For instance, second adhesive 916 may follow an inner perimeter or surface area of securement pad 908. Second adhesive 916 may extend radially outward from a center of securement pad 908 and/or wearable medical device 904 alongside ends 928. In some embodiments, securement pad 908 may include relief areas 920. Relief areas 920 may be one or more parts of securement pad 908 that may be adhesive free. Relief areas 920 may be openings within securement pad 908, such that a user's skin may avoid contact with securement pad 908 at relief areas 920.

Relief areas 920 may be circular, rectangular, square, or other shapes. In some embodiments, securement pad 908 may have two or more relief areas 920. For instance and without limitation, securement pad 908 may have three or more relief areas 920. In embodiments where securement pad 908 may have multiple relief areas 920, each relief area 920 of a plurality of relief areas 920 may have the same dimensions. Each relief area 920 of a plurality of relief areas 920 may be positioned at equal distance to each other. For instance, each relief area 920 of a plurality of relief areas 920 may be positioned about 1 mm to about 5 mm away from each other. Relief areas 920 may be rounded rectangular openings within securement pad 908, in some embodiments. Relief areas 920 may be positioned at a central point of securement pad 908 and/or wearable medical device 904. Relief areas 920 may assist in removal of securement pad 908 and/or wearable medical device 904 from a user's skin. Second adhesive 916 may have a geometry mirroring that of securement pad 908 except for relief areas 920 and insertion adhesive 924. Insertion adhesive 924 may be a circular, rectangular, or other shaped portion of second adhesive 916 that may surround, partially or completely, insertion hole 936 of wearable medical device 904. Insertion hole 936 may be a portion of wearable medical device 904 that may deploy an insertion mechanism, such as a needle and/or cannula, sensing element, or other device. Insertion hole 936 may be surrounded by both second adhesive 916 via insertion adhesive 924 and first adhesive 912 which may be disposed within and/or outside of insertion adhesive 924. Insertion adhesive 924 and/or insertion hole 936 may be positioned at a corner of wearable medical device 904. For instance and without limitation, insertion hole 936 may be positioned at a center of an end 928 of securement pad 908. Insertion adhesive 924 may have a thickness greater than first adhesive 912 and/or second adhesive 916. In some embodiments, insertion adhesive 924 may be a third adhesive separate from first and second adhesives 912 and 916.

Referring still to FIG. 9, first adhesive 912 may be an adhesive having a stronger immediate bond strength than second adhesive 916. First adhesive 912 may provide an outer perimeter coverage of securement pad 908 while second adhesive 916 may provide an inner area coverage of securement pad 908. First adhesive 912 may be an acrylic-based, rubber-based, or silicone-based adhesive. Likewise, second adhesive 916 may be an acrylic-based, rubber-based, or silicone-based adhesive. Thickness and/or tackiness of first adhesive 912 and/or second adhesive 916 may be adjusted for various skin types. In some embodiments, first adhesive 912 First adhesive 912 may have a greater or lesser thickness than second adhesive 916, and vice versa. First adhesive 912 may have a greater or lesser tack than second adhesive 916, and vice versa. In some embodiments, second adhesive 916 may be thicker than first adhesive 912. First adhesive 912 may have a greater area of coverage and/or contact with a user's skin than second adhesive 916. In some embodiments, securement pad 908 may have a ratio of one or more adhesives to one or more other adhesives. For instance and without limitation, securement pad 908 may be disposed with a ratio of first adhesive 912 to second adhesive 916 of about 1:1, 1.5: 1, 2:1, 2.5: 1, 3:1, 3.5: 1, 4: 1, 4.5: 1, 5:1, or greater than 5:1. Second adhesive 916 may be an adhesive that has an increasing bond strength over time. Bond strengths of first and second adhesive 912 and 916 may be depicted below with reference to FIG. 10.

Referring now to FIG. 10, a graph 1000 illustrating bond strengths of adhesives is shown. Graph 1000 shows multiple lines with a y-axis representing bond strength or adhesion to skin with an x-axis representing time in days. In some embodiments, bond strength may be measured over a span of about 6 to 12 days. Bond strength may be measured in pounds (lbs), newtons (N) or other measurements. In some embodiments, bond strength may be measured as force per area, for instance and without limitation as a pressure per square inch (PSI), newtons per meter squared (Pa), or other values. Line 1016 represents a maximum bond strength that if reached makes removing an adhesive pad difficult. Line 1010 shows a minimum bond strength that if reached makes an adhesive too weak to secure to a wearable medical device and/or user's skin. Graph 1000 shows first adhesive 1004, which may be first adhesive 912 as described above with reference to FIG. 9. As shown, first adhesive 1004 may have a high initial bond strength that may decrease over time. In some embodiments, first adhesive 1004 may have an approximately linear decay of bond strength over time. Graph 1000 shows second adhesive 1008, which may be second adhesive 916 as described above with reference to configurations. FIG. 9. Second adhesive 1008 may have a lower initial bond strength compared to first adhesive 1004. In some embodiments, second adhesive 1008 may have an initial bond strength that may be lower than minimum bond strength 1020. Over time, second adhesive 1008 may increase in a bond strength. In some embodiments, a bond strength of second adhesive 1008 may be equal to a bond strength of first adhesive 1004 at a point in time. Second adhesive 1008 may increase in bond strength and surpass a decreasing bond strength of first adhesive 1004. A combined bond strength 1012 of first adhesive 1004 and second adhesive 1008 may remain approximately linear over time. For instance, combined bond strength 1012 may have an initial bond strength slightly higher than an initial bond strength of first adhesive 1004 and may dip slightly over time compared to the dip in bond strength of first adhesive 1004 over time. A combination of first adhesive 1004 and second adhesive 1008 may allow a relatively stable bond strength of combined bond strength 1012 over time.

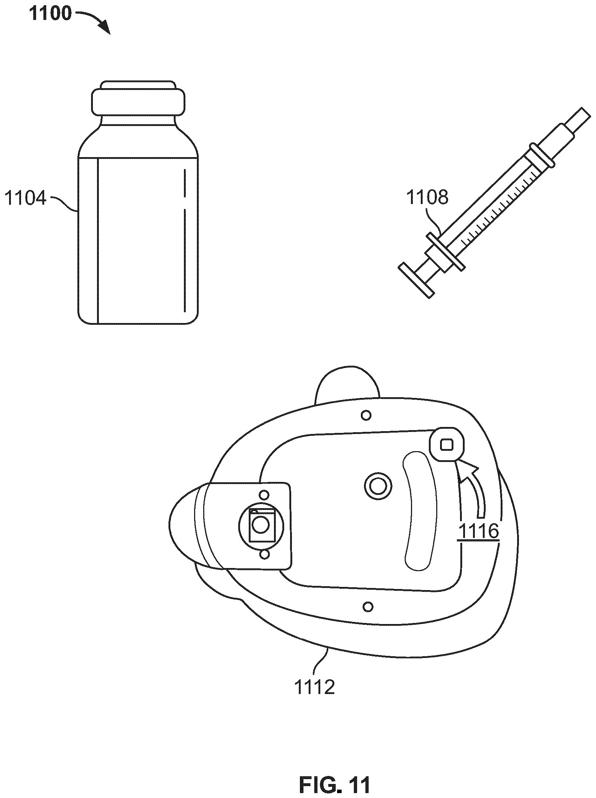

Referring now to FIG. 11, a system 1100 for filling a medicament reservoir is shown. System 1100 may include vial 1104. A "vial" as used in this disclosure is any object that contains a fluid. The vial 1104 may include, without limitation, a glass, plastic, or other vial. In some embodiments, the vial 1104 may have a volume of about 10 mL. In other embodiments, the vial 1104 may have a volume of greater than or less than about 10 mL. The vial 1104 may contain a medicament, such as, but not limited to, medicaments with close relation to diabetes care such as insulin, glucagon, pramlintide, or others, as well as other medicaments such as saline, morphine, and/or other compositions. The vial 1104 may contain a concentration of insulin, such as, without limitation, U-100, U-500, and the like. In some embodiments, the vial 1104 may contain a type of insulin, such as, but not limited to, fast acting, slow acting, and/or other types of insulin. The vial 1104 may include a colored insulin formulation. A colored insulin formulation may include a combination of insulin and one or more food colorings, or through the addition of trace amounts of non-biologically active materials, through delivery methods such as nanoparticles. Insulin contained in the vial 1104 may be colored coded. For instance, and without limitation, insulin contained in the vial 1104 may be colored purple, which may represent a fast acting, low concentration of insulin. In another non-limiting example, insulin contained in the vial 1104 may be colored yellow, which may represent a slow acting, high concentration of insulin. In some embodiments, the vial 1104 may have a colored label or other visual indicator that may correspond to a type of medicament. For instance, the vial 1104 may have a purple sticker representing a fast acting, low concentration of insulin, without limitation. In another embodiment, the vial 1104 may have a yellow sticker that may represent a slow acting, high concentration of insulin. The vial 1104 may have any colored label, sticker, and/or insulin formulation, such as, without limitation, red, green, blue, yellow, and/or any combination thereof.

Still referring to FIG. 11, in some embodiments, system 1100 may include syringe 1108. The syringe 1108 may include a needle that may be configured to puncture a top surface of the vial 1104. The syringe 1108 may have a volume of about 20 mL. In other embodiments, the syringe 1108 may have a volume of greater than or less than 20 mL. The syringe 1108 may be configured to retrieve a medicament from the vial 1104. For instance, the syringe 1108 may be configured to retrieve about 10 mL of an insulin contained in the vial 1104, without limitation. In other embodiments, the syringe 1108 may be configured to retrieve more than or less than about 10 mL of a medicament contained in the vial 1104.

The syringe 1108 may be configured to expel an amount of a medicament into the wearable medical device 1112. The wearable medical device 1112 may include, without limitation, an insulin pump. The wearable medical device 1112 may include port 1116. The port 1116 may be a component of the wearable medical device 1112 that may be configured to receive and/or interface with an end of the syringe 1108. For instance, the syringe 1108 may fit a needle end into the port 1116. The port 1116 may comprise a port marker. The port marker may mark which medicament is to be introduced into the port. The port 116 may be color coded to match a type of medicament in the vial 1104. Accordingly, the port marker may be a color port marker. For instance, the vial 1104 may contain a purple colored medicament, the syringe 1108 may have a purple sticker or other label, and the port 1116 may be colored purple. The purple coloring in this non-limiting example may represent a fast acting, low concentration of insulin. In another non-limiting example, the vial 1104 may contain a yellow colored medicament, the syringe 1108 may have a yellow sticker or other label, and the port 1116 may be colored yellow. The yellow color in this non-limiting example may represent a slow acting, high concentration of insulin. One of ordinary skill in the art, upon reading this disclosure, will appreciate the many various colors that may be used in the system 1100. Accordingly, a kit of parts may be provided. The kit of parts comprising a wearable drug delivery device comprising a reservoir in fluid communication with a port; and a reservoir filling kit. The reservoir filling kit may comprise a filling kit marker, and the port may comprise a port marker, wherein the filling kit marker and the port marker correspond to one another. The reservoir filling kit may comprise the vial and the syringe. As mentioned above, the vial may comprise the filling kit marker. In some embodiments, the syringe may comprise a syringe marker that corresponds to the port marker and filling marker. In some embodiments, the port marker and the filling kit marker may be colored markers, wherein the port marker and the filling kit marker have the same color. In some embodiments, the syringe marker may also be a color marker with the same color as the filling kit marker. In some embodiments, the port marker is arranged around or next to an opening of the port.

Marking the ports may be in particular beneficial for drug delivery devices that can provide more than one medication, for example, fast-acting and slow-acting insulin. Accordingly, a wearable drug delivery device may comprise a first reservoir in fluid communication with a first opening of a first reservoir port comprising a first port marker, and a second reservoir in fluid communication with a second opening of a second reservoir port comprising a second port marker, wherein the first port marker and the second port marker are different. The wearable drug delivery device may be provided as a kit of parts. The kit of parts may comprise the aforementioned wearable drug delivery device, a first filling kit comprising a first filling kit marker corresponding to the first port marker, and a second filling kit comprising a second filling kit marker corresponding to the second port marker. Further, the first filling kit may comprise a first vial comprising the first filling kit color marker, and the second filling kit may comprise a second vial comprising the second filling kit color marker. The kit of parts may additionally comprise a syringe comprising two graduations, wherein the first graduation comprises graduation markers in the color of the first port color marker and the second graduation comprises graduation markers in the color of the second port color marker.

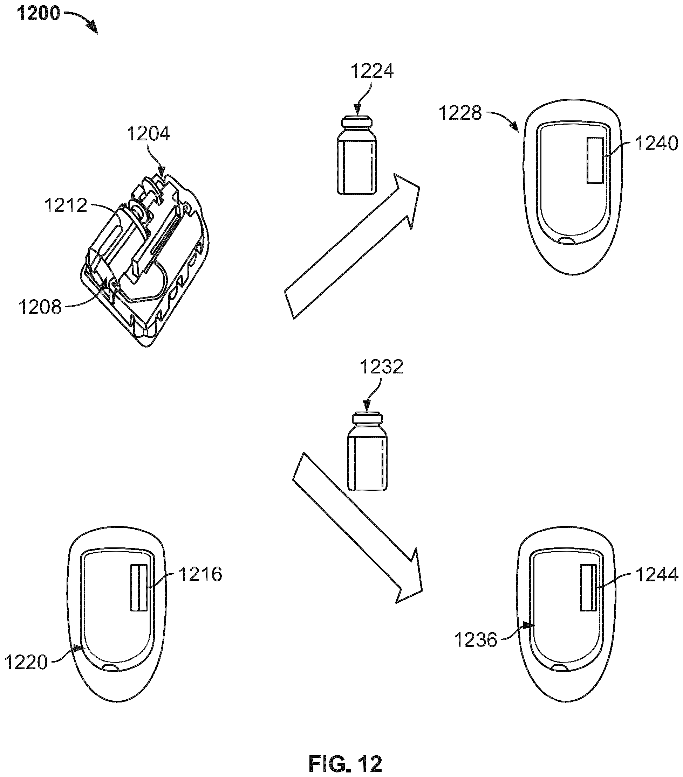

Referring now to FIG. 12, a system 1200 for medicament filling is presented. The system 1200 may include wearable medical device 1204. The wearable medical device 1204 may include, without limitation, an insulin pump. The wearable medical device may include reservoir 1208. The reservoir 1208 may be configured to contain or otherwise hold a liquid. For instance, the reservoir 1208 may be configured to contain a liquid medicament, such as, but not limited to, insulin. The reservoir 1208 may include label 1212. The label 1212 may be a sticker or other label that may be positioned on an exterior or interior of the reservoir 1208.

The label 1212 may be visible from the viewing window 1216 of the adhesive patch 1220 that may house the wearable medical device 1204. The viewing window 1216 of the adhesive patch 1220 may be an uncovered portion of the adhesive patch 1220 that may allow the reservoir 1208 to be visible outside the adhesive patch 1220. In some embodiments, the viewing window 1216 may be positioned on a top right surface of the adhesive patch 1220. In other embodiments, the viewing window 1216 may be positioned on a top left, bottom left, bottom right, central, or other position of the adhesive patch 1220. The label 1212 may align with a side of the viewing window 1216. For instance, the label 1212 may be positioned on a left, right, top, or bottom side of the viewing window 1216, without limitation.

A user or other individual may fill the reservoir with the correct medicament 1224, which may be colored the same as the label 1212. For instance, the label 1212 and the correct medicament 1224 may each be colored purple. In some embodiments, upon filling the reservoir 1208 with the correct medicament 1224, the viewing window 1216 may appear as a solid color. For instance, the label 1212 and the correct medicament 1224 may both be colored purple, which may make the viewing window 1216 appear as a solid purple rectangle or other shape, such as in the uniform viewing window 1240 of the correctly filled device 1228.

In some embodiments, a user or other individual may fill the reservoir 1208 with the incorrect medicament 1232. The incorrect medicament 1232 may include a colored composition, such as insulin. In some embodiments, the incorrect medicament 1232 may be colored differently than the label 1212 of the viewing window 1216. As a non-limiting example, the incorrect medicament 1232 may be colored yellow while the label 1212 may be colored purple. The reservoir 1208 may be filled with the incorrect medicament 1232, which may contrast against the label 1212, appearing as two distinct colored lines in the viewing window 1216 of incorrectly filled device 1236. The incorrectly filled device 1236 may have a contrasting viewing window 1244. The contrasting viewing window 1244 may display two or more colors, which may be indicative of an incorrectly filed reservoir of the incorrectly filled device 1236. Colors of the label 1212, correct medicament 1224, and/or the incorrect medicament 1232 may represent a type of drug, such as insulin. For instance, the correct medicament 1224 and the label 1212 may both be colored purple to represent a fast acting, low concentration insulin while the incorrect medicament 1232 may represent a slow acting, high concentration of insulin. The uniform viewing window 1240 and/or the contrasting viewing window 1244 may alert or otherwise display to a user a correct or incorrect filling of the reservoir 1208 of the wearable medical device 1204. Accordingly, a wearable drug delivery device may comprise a first reservoir in fluid communication with a first reservoir port, wherein the first reservoir comprises a first viewing window, wherein a first viewing window label is arranged around or next to the first viewing window. Again, the viewing windows may be in particular beneficial for wearable drug delivery devices allowing the delivery of different medication to assess whether each medication was applied to the intended reservoir. Accordingly, in some embodiments, the wearable drug delivery device may comprise a second reservoir in fluid communication with a second reservoir port, wherein the second reservoir comprises a second viewing window, wherein a second viewing window label is arranged around or next to the first viewing window; and wherein the first viewing window label and the second viewing window label are different, in particular wherein the first viewing window label and the second viewing window label have different colors. The aforementioned drug delivery device may be part of a kit of parts. The kit of parts may comprise the drug delivery device and additionally comprises a first vial comprising a first fluid drug and a second vial comprising a second fluid drug, wherein the first vial has a first vial marker corresponding to the first viewing window label, and wherein the second vial marker has a second vial marker corresponding to the second viewing window label; and/or, wherein the first fluid drug has a color corresponding to the color of the first viewing window label and wherein the second fluid drug has a color corresponding to the second viewing window label.

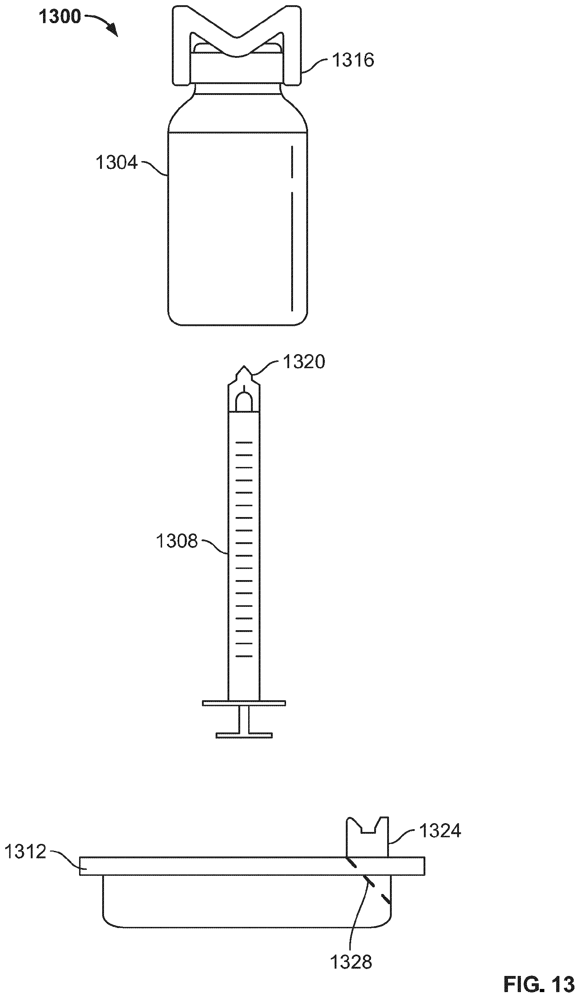

Referring now to FIG. 13, another embodiment of a system 1300 for medicament filling is presented. System 1300 may include vial 1304, syringe 1308, and/or wearable medical device 1312, each of which may be as described above with reference to FIG. 9. The vial 1304 may have adapter 1316. The adapter 1316 may be comprised of a polymer, plastic, rubber, and/or other material. In some embodiments, the adapter 1316 may be shaped as, without limitation, a convex, concave, square, rectangular, octangular, and/or other shape. In an embodiment, the adapter 136 may be shaped as an "M" or other shape. The adapter 1316 may be positioned and/or placed on top of the vial 1004. The adapter 1316 may secure to a top of the vial 1004 through an adhesive, tension of a rubber opening of the adapter 1316, and/or other methods, without limitation. For instance, and without limitation, the adapter 1316 may have a rubber opening on a bottom portion, which may be circular to adhere to a top of the vial 1304.

The syringe 1308 may have a key adapter 1320. The key adapter 1320 may be comprised of a rubber, polymer, plastic, and/or material, without limitation. In some embodiments, the key adapter 1320 may be shaped as, but not limited to, a convex, concave, square, rectangular, circular, octagonal, and/or other shape. The key adapter 1320 may have a protruding shape that may fit into a concave area of the adapter 1316. As a non-limiting example, the key adapter 1320 may be shaped as an upside down "V" and the adapter 1316 may be shaped as a right side up "V", which may allow the key adapter 1320 to insert into the concave surface of the adapter 1316. The key adapter 1320 and/or the adapter 1316 may correspond to a specific type of medicament. For instance, a shape of the key adapter 1320 and a corresponding shape of the adapter 1316 may correspond to a fast acting, low concentration insulin. In some embodiments, a shape of the key adapter 1320 may correspond to a different medicament type than a shape of the adapter 1316, and vice versa. For instance, the shape of the key adapter 1320 may be shaped as a circle and the shape of the adapter 1316 may be shaped as a square, which may prevent the syringe 1308 from being able to deliver a medicament into the reservoir 1304. In some embodiments, the adapter 1316 and/or the key adapter 1320 may be colored as the same color, which may represent a type of medicament, such as, without limitation, a fast acting, low concentration insulin or a slow acting, high concentration insulin.

Still referring to FIG. 13, system 1300 may include wearable medical device 1324, which may be as described above with reference to FIG. 9. The wearable medical device 1324 may include device adapter 1328. The device adapter 1328 may be shaped to allow the key adapter 1320 to enter a pump fill port of the wearable medical device 1324. For instance, and without limitation, the device adapter 1328 may be shaped the same as the adapter 1316, allowing for the syringe 1308 to expel a medicament into a pump fill port of the wearable medical device 1324. In some embodiments, the device adapter 1328 may be color coded along with the adapter 1316 and/or the key adapter 1320. For instance, the device adapter 1328, adapter 1316, and/or key adapter 1320 may all be colored purple, blue, yellow, and the like, without limitation. A color may represent a type of medicament, such as a concentration of insulin, short acting insulin, fast acting insulin, and/or other medicaments, without limitation.

The device adapter 1328 may include a latch 1332. The latch 1332 may be configured to block off a port of the wearable medical device 1324, such as a pump fill port. In some embodiments, the latch 1332 may be a one time use latch. In other embodiments, the latch 1332 may be reusable. The latch 1332 may prevent the syringe 1308 from filling a reservoir of the wearable medical device 1324 after the device adapter 1328 has been removed. As a non-limiting example, a user may fill a reservoir of the wearable medical device 1324 through the device adapter 1328 and/or the key adapter 1320 of the syringe 1308. After the wearable medical device 1324 has been filled, a user may remove the device adapter 1328, to enable the users to wear the device without the adapter. This mechanism may also cause the latch 1332 to shut off access to a pump fill port of the wearable medical device 1324 and prevent the user or others from filing the pump with a non-compatible medicament, by a plastic or other latch that automatically moves to cover the fill port due to the force of the removal of the device adapter.

Referring now to FIG. 14, another embodiment of a medicament filling system 1400 is presented. The system 1400 may include vial 1404, syringe 1408, adapter 1412, key adapter 1416, wearable medical device 1420, device adapter 1424 and/or latch 1428 may be the same as described above with reference to FIG. 11.

The adapter 1412 may be shaped concavely, with a protruding end, and the key adapter 1416 may be shaped convexly, pointing inwards, in some embodiments, The adapter 1412 and the key adapter 1416 may interface by matching the concave surface of the key adapter 1416 with the convex surface of the adapter 1412. The device adapter 1424 may also be shaped concavely, allowing for an interface with the key adapter 1412. In some embodiments, the adapter 1412, key adapter 1416, and/or device adapter 1424 may share a color, such as, without limitation, yellow. A shared color of the adapter 1412, key adapter 1416, and/or the device adapter 1424 may correspond to a type of medicament, such as, without limitation, a slow acting, high concentration of insulin. In some embodiments, the wearable medical device 1420 may have latch 1428. The latch 1428 may be as described above with reference to FIG. 11. The latch 1428 may be configured to prevent further delivery of a medicament through the syringe 1408. For instance, in an embodiment, after the device adapter 1424 is removed, the latch 1428 may snap into a position blocking off any entrance to a pump fill port of the wearable medical device 1420. The aforementioned adapters may be in particular beneficial for wearable drug delivery devices being able to deliver two different medicaments, to ensure that the intended reservoir is filled. Accordingly, a wearable drug delivery device may comprise a first reservoir in fluid communication with a first reservoir port, wherein the first reservoir port comprises a first port adapter; and a second reservoir in fluid communication with a second reservoir port, wherein the second reservoir port comprises a second port adapter. The first port adapter and second port adapter may be configured to allow entry of a syringe with a corresponding key adapter. The aforementioned wearable drug delivery device may be provided as a kit of parts. The kit of parts may additionally comprise a first syringe comprising a first syringe key adapter configured to fit with the first port adapter, such that the first syringe can be introduced into the first port and wherein the first syringe key adapter is additionally configured to not fit with the second port adapter such that the first syringe cannot be introduced in the second port. The kit of parts may additionally comprise a second syringe comprising a second syringe key adapter configured to fit with the second port adapter, such that the second syringe can be introduced into the second port and wherein the second syringe adapter is additionally configured to not fit with the first port adapter such that the second syringe cannot be introduced in the first port.

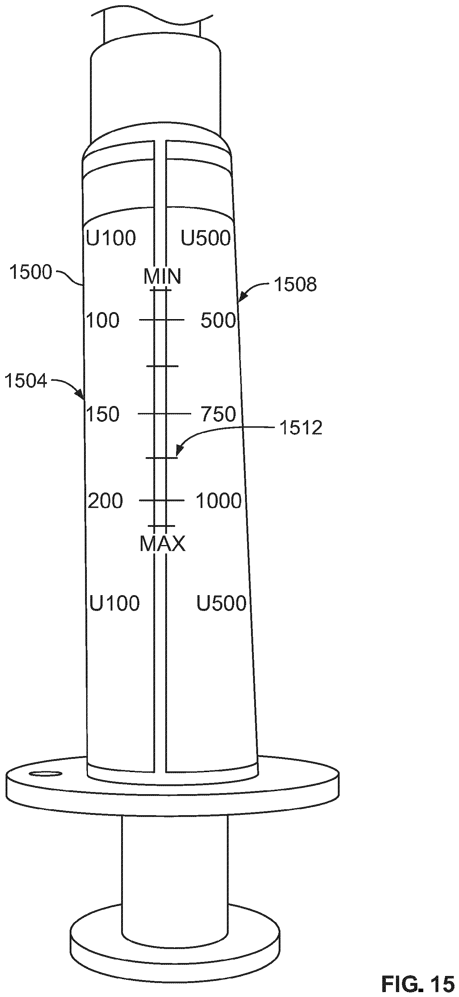

Referring now to FIG. 15, a dual graduation syringe 1500 is presented. The syringe 1500 may be any type of syringe, such as, but not limited to, luer-lock, luer-slip, insulin, safety, and/or other syringe types. In an embodiment, the syringe 1500 may be an insulin syringe. The syringe 1500 may have a volume, such as, but not limited to, about 10 mL. In other embodiments, the syringe 1500 may have a volume greater than or less than about 10 mL, without limitation. The syringe 1500 may have first graduation 1504. The first graduation 1504 may include one or more measuring indicators for a type of insulin. For instance, and without limitation, the first graduation 1504 may correspond to the U100 concentration of insulin. The first graduation 1504 may have one or more characters, numbers, and the like along indicators 1512. The indicators 1512 may include, without limitation, dots, lines, stripes, and the like. Each indicator of the indicators 1512 may correspond to a volume of medicament contained in the syringe 1500. In some embodiments, the indicators 1512 may correspond to a dosage of a medicament, such as, without limitation, insulin. The indicators 1512 may include 7 horizontal lines. In other embodiments, the indicators 1512 may include less than or greater than 7 horizontal lines. The indicators 1512 may include a minimum indicator. A minimum indicator may be a horizontal line at a top of the syringe 1500 that may correspond to a minimum dosage of a medicament. In some embodiments, the indicators 1512 may include a maximum indicator, which may correspond to a maximum dosage of a medicament. The indicators 1512 may vary in length, thickness, and/or other dimensions. For instance, and without limitation, the indicators 1512 may include a repeating pattern of a long horizontal line followed by a short horizontal line. A long horizontal line of the indicators 1512 may represent an interval of a dosage of a medicament and a short horizontal line of the indicators 1512 may represent a halfway point between two intervals of a dosage. In some embodiments, each indicator of the indicators 1512 may be spaced about 2 mm apart. In other embodiments, each indicator of the indicators 1512 may be spaced greater than or less than about 2 mm apart. In some embodiments, each long horizontal line of the indicators 1512 may be spaced apart by about 4 mm and each short horizontal line of the indicators 1512 may be spaced apart by about 4 mm. In other embodiments, each short horizontal line of the indicators 1512 may be spaced greater than or less than about 4 mm and each long horizontal line of the indicators 1512 may be spaced greater than or less than about 4 mm, without limitation.

The first graduation 1504 may correspond to dosages of U100 insulin and may include one or more numbers or text corresponding to the U100 insulin type. For instance, the first graduation 1504 may include a plurality of numbers and text along a left side of the syringe 1500 while the second graduation 1508 may include a plurality of numbers and text along a right side of the syringe 1500, or vice versa, without limitation. The first graduation 1504 may include a label at a bottom and/or top of the syringe 1500, such as text reading "U100". The first graduation 1504 may have one or more numbers aligned with longer horizontal lines of the indicators 1512. For instance, at each long horizontal line of the indicators 1512, intervals of 50 starting from 100 may be shown. As a non-limiting example, the first graduation 1504 may have a first number of 100 displayed next to a first long horizontal line of the indicators 1512, a second number of 150 displayed at a second horizontal line of the indicators 1512, and a third number of 200 displayed at a third indicator of the indicators 1512. Each number of the first graduation 1504 may correspond to an insulin dosage amount of a U100 concentration, such as, but not limited to, 100, 150, 200, and the like. In some embodiments, at short horizontal lines of the indicators 1512, the first graduation 1504 may display a number in between dosages, such as, but not limited to, halfway between a first dosage number and a second dosage number. As a non-limiting example, the first graduation 1504 may display a first number of 125 at a first horizontal line of the indicators 1512 and a second number of 175 at a second horizontal line of the indicators 1512.

Still referring to FIG. 15, the second graduation 1508 may include one or more numbers corresponding to dosages of a U500 concentration of insulin. The second graduation 1508 may include a label at a top and/or bottom of the syringe 1500 that may display a concentration of insulin, such as, without limitation, "U500". The second graduation 1508 may a number representing a dose of U500 insulin at each horizontal line of the indicators 1512. As a non-limiting example, the second graduation 1508 may include a number of 500 at a first long horizontal line of the indicators 1512, a number of 750 at a second long horizontal line of the indicators 1512, and a number of 1000 at a third long horizontal line of the indicators 1512. In some embodiments, the second graduation 1508 may display intervals of dosages, such as, but not limited to, 625, 1,250, and the like, which may be displayed at short horizontal lines of the indicators 1512. The first gradation 1504 and second gradation 1508 within the syringe 1500 may be designed to facilitate a user's determination of whether the correct medicament type was filled in the syringe. For instance, the color of the U100 insulin solution and the gradations 1504 and 1508 may be both designed such that the gradations may merge in their visibility with the solution. In comparison, if U500 insulin with a different color, a clear U500 solution may be utilized, the gradations may continue to be highly visible after being filled, providing a clear indication to the user that an incorrect medicament was used to fill the syringe.

FIG. 16 illustrates an exemplary medicament delivery system operable to implement the examples disclosed herein. In some examples, the medicament delivery system 1600 is suitable for delivering a liquid medicament, such as insulin to a user in accordance with the disclosed embodiments. The medicament delivery system 1600 may include a wearable medicament delivery device 1602, a controller 1604 and an analyte sensor(s) 1606.

The wearable medicament delivery device 1602 may be a wearable device that is worn on the body of the user. The wearable medicament delivery device 1602 may be directly coupled to a user (e.g., directly attached to a body part and/or skin of the user via an adhesive, or the like). In an example, a surface of the wearable medicament delivery device 1602 may include an adhesive to facilitate attachment to the skin of a user.

The wearable medicament delivery device 1602 may include a processor 1614. The processor 1614 may be implemented in hardware, software, or any combination thereof. The processor 1614 may, for example, be a microprocessor, a logic circuit, a field programmable gate array (FPGA), an application specific integrated circuit (ASIC) or a microprocessor coupled to a memory. The processor 1614 may maintain a date and time as well as be operable to perform other functions (e.g., calculations or the like). The processor 1614 may be operable to execute a control application 1626 stored in the memory 1612 that enables the processor 1614 to direct operation of the wearable medicament delivery device 1602. The control application 1626 may control insulin delivery to the user per an ADD control approach as describe herein. For example, the control application 1626 may be an ADD algorithm. The memory 1612 may hold settings 1624 for a user, such as ADD algorithm settings, such as maximum insulin delivery, insulin sensitivity settings, total daily insulin (TDI) settings and the like. The memory may also store other data 1629, such as ketone values, total daily insulin values, glucose measurement values from analyte sensor(s) 1606 or controller 1604, insulin dosage amounts (both basal and bolus) and the like from previous minutes, hours, days, weeks, or months. The analyte sensor(s) 1606 may be operable to collect physiological condition data, such as ketone values, ketone values with a time stamp, glucose measurement values (also referred to herein as "blood glucose values" or "blood glucose"), glucose measurement values and a timestamp, both ketone values and glucose values that may be shared with the wearable medicament delivery device 1602, the controller 1604 or both. In an additional example, the analyte sensor(s) 1606 may include multiple sensors, such as a continuous glucose monitor 1686 and a ketone sensor(s) 1696. In a further example, the wearable medicament delivery device 1602 may optionally include a continuous glucose monitor 1686A and a ketone sensor 1696A, which may be removably incorporated or fully integrated within the wearable medicament delivery device 1602. For example, the continuous glucose monitor 1686A and the ketone sensor 1696A may be incorporated in one or more housings 1602H of the wearable medicament delivery device 1602. Note that ketones may also be detected using a breath sensor (which is not shown but may be incorporated in the controller 1604) or urine content sensor and stored in the respective memories 1618 and 1612 via a user input of the ketone levels; however, a subcutaneous ketone sensor(s) gives more accurate information and is more continuous. As described herein, the MDA and ADD system is described based on receiving ketone values received subcutaneously. Use of a ketone breath sensor or urine sensor as part of or in addition to the analyte sensor(s) 1606 may delay receipt of the ketone values and modifications to the system 1600 may be made.

For example, the communication circuitry 1642 of the wearable medicament delivery device 1602 may be operable to communicate with the analyte sensor(s) 1606 and the controller 1604 as well as the devices 1630, 1633 and 1634. The communication circuitry 1642 may be operable to communicate via Bluetooth, cellular communication, near field communication (NFC) and/or other wireless protocols. While not shown, the memory 1612 may include both primary memory and secondary memory. The memory 1612 may include random access memory (RAM), read only memory (ROM), optical storage, magnetic storage, removable storage media, solid state storage or the like.

The wearable medicament delivery device 1602 may include a reservoir 1620. The reservoir 1620 may be operable to store liquid drugs, medicaments, or therapeutic agents suitable for automated delivery, such as, insulin, glucagon-like peptide-1 (GLP-1) receptor agonist, pramlintide, glucose-dependent insulinotropic polypeptide (GIP), other hormones, or co-formulations of two or more of glucagon, GLP-1, pramlintide, and insulin; as well as pain relief drugs, such as opioids or narcotics (e.g., morphine, or the like), methadone, blood pressure medicines, chemotherapy drugs, fertility drugs, or the like.

A fluid path to the user may be provided via tubing and a needle/cannula (not shown). The fluid path may, for example, include tubing coupling the wearable medicament delivery device 1602 to the user (e.g., via tubing coupling a needle or cannula to the reservoir 1620). The wearable medicament delivery device 1602 may be operable based on control signals from the processor 1614 to expel the liquid drugs, medicaments, or therapeutic agents, such as insulin, from the reservoir 1620 to deliver doses of the drugs, medicaments, or therapeutic agents, such as the insulin, to the user via the fluid path. The processor 1614 may be operable to cause insulin to be expelled from the reservoir 1620.

There may be one or more communications links 1628 with one or more devices physically separated from the wearable medicament delivery device 1602 including, for example, a controller 1604 of the user and/or a caregiver of the user and/or a sensor(s) 1606. The communication links 1628 may include any wired or wireless communication link operating according to any known communications protocol or standard, such as Bluetooth^{®}, NFC, Wi-Fi, a near-field communication standard, a cellular standard, or any other wireless protocol. The analyte sensor(s) 1606 may communicate with the wearable medicament delivery device 1602 via a wireless communication link 1631 and/or may communicate with the controller 1604 via a wireless communication link 1637.

The wearable medicament delivery device 1602 may also include a user interface 1616, such as an integrated display device for displaying information to the user, and in some embodiments, receiving information from the user. For example, the user interface 1616 may include a touchscreen and/or one or more input devices, such as buttons, knob(s), or a keyboard that enable a user to provide an input.

In addition, the processor 1614 may be operable to receive data or information from the analyte sensor(s) 1606 as well as other devices that may be operable to communicate with the wearable medicament delivery device 1602.

The wearable medicament delivery device 1602 may interface with a network 1608. The network 1608 may include a local area network (LAN), a wide area network (WAN) or a combination therein. A computing device 1632 may be interfaced with the network, and the computing device may communicate with the insulin delivery device 1602. The computing device 1632 may be a healthcare provider device through which a user's controller 1604 may interact to obtain information, store settings and the like. The ADD algorithm operating, as or in cooperation with, the control application 1620 may present a graphical user interface on the computing device 1632 enabling the input and presentation of information related to the ADD algorithm and the example techniques and processes described herein.

The medicament delivery system 1600 may include an analyte sensor(s) 1606 for sensing the levels of one or more analytes of a user. The analyte levels may be used as physiological condition data and be sent to the controller 1604 and/or the wearable medicament delivery device 1602. The sensor(s) 1606 may be coupled to the user by, for example, adhesive or the like and may provide information or data on one or more medical conditions and/or physical attributes of the user. The sensor(s) 1606 may be a continuous glucose monitor (CGM), ketone sensor, or another type of device or sensor(s) that provides glucose measurements and/or ketone measurements. The sensor(s) 1606 may be physically separate from the wearable medicament delivery device 1602 or may be an integrated component thereof. The sensor(s) 1606 may provide the processor 1614 and/or processor 1619 with physiological condition data indicative of measured or detected glucose levels of the user. The information or data provided by the sensor(s) 1606 may be used to modify a medicament delivery schedule and thereby cause the adjustment of medicament delivery operations of the wearable medicament delivery device 1602.

The medicament delivery system 1600 may also include the controller 1604. In the depicted example, the controller 1604 may include a processor 1619 and a memory 1618. The controller 1604 may be a special purpose device, such as a dedicated personal diabetes manager (PDM) device. The controller 1604 may be a programmed general-purpose device that is a portable electronic device, such as any portable electronic device, smartphone, smartwatch, fitness device, tablet or the like including, for example, a dedicated processor, such as processor, a micro-processor or the like. The controller 1604 may be used to program or adjust operation of the wearable medicament delivery device 1602 and/or the sensor(s) 1606. The processor 1619 may execute processes to control the delivery of the medicament, drug, or therapeutic agent to the user for the purpose of managing a user's blood glucose and/or ketone levels. The processor 1619 may also be operable to execute programming code stored in the memory 1618. For example, the memory 1618 may be operable to store a control application 1620, such as an ADD algorithm for execution by the processor 1619. The control application 1620 may be responsible for controlling the wearable medicament delivery device 1602, including the automated delivery of medicament, a drug, or therapeutic agent based on recommendations and instructions from the ADD algorithm, such as those recommendations and instructions described herein.

The memory 1618 may store one or more applications, such as control application 1620, and settings 1621 for the medicament delivery device 1602 like those described above. In addition, the memory 1618 may be operable to store other data 1639 and/or computer programs, such as medicament delivery history, glucose measurement values over a period of time, total daily insulin values, ketone values, and the like. For example, the memory 1618 is coupled to the processor 1619 and operable to store programming instructions, such as the control application 1620 and settings 1621, and data, such as other data 1639, related to a glucose of a user and/or data related to an amount of insulin expelled by the wearable medicament delivery device 1602.

The controller 1604 may include a user interface (UI) 1623 for communicating with the user. The user interface 1623 may include a display, such as a touchscreen, for displaying information. The touchscreen may also be used to receive input when it is a touch screen. The user interface 1623 may also include input elements, such as a keyboard, button, knob or the like. In an operational example, the user interface 1623 may include a touchscreen display (including a display and user input circuitry, such as touch sensitive circuits and the like) controllable by the processor 1619 and be operable to present a graphical user interface and receive inputs via the user input circuitry, the touchscreen display may be operable to generate a signal indicative of the respective diet types, number of days, diet cycles, keto days, location information, calendar information, or the like. The touchscreen display, under control of the processor 1619, may be operable to, in response to the received input, generate a response to a user's drug treatment regimen as well as cause the presentation of prompts on a graphical user interface. The graphical user interfaces discussed herein may be generated by the processor 1619 of the controller 1604 and be presented on the UI 1623.

The controller 1604 may interface via a wireless communication link of the wireless communication links 1628 with a network, such as a LAN or WAN or combination of such networks that provides one or more servers or cloud-based services 1610 via communication circuitry 1622. The communication circuitry 1622, which may include transceivers 1627 and 1625, may be coupled to the processor 1619. The communication circuitry 1622 may be operable to transmit communication signals (e.g., command and control signals) to and receive communication signals (e.g., via transceivers 1627 or 1625) from the wearable medicament delivery device 1602 and the analyte sensor(s) 1606. In an example, the communication circuitry 1622 may include a first transceiver, such as 1625, that may be a Bluetooth transceiver, which is operable to communicate with the communication circuitry 1622 of the wearable medicament delivery device 1602, and a second transceiver, such as 1627, that may be a cellular or Wi-Fi transceiver operable to communicate via the network 1608 with computing device 1632 or with cloud-based services 1610.

The cloud-based services 1610 may be operable to receive and store user history information, such as glucose measurement values over a set period of time (e.g., days, months, years), a medicament delivery history that includes insulin delivery amounts (both basal and bolus dosages) and insulin delivery times, types of insulin delivered, indicated meal times, glucose measurement value trends or excursions or other user-related diabetes treatment information, specific factor settings including default settings, present settings and past settings, or the like.

Other devices, like smart accessory device 1630 (e.g., a smartwatch or the like), fitness device 1633 and other wearable device 1634 may be part of the medicament delivery system 1600. These devices may communicate with the wearable medicament delivery device 1602 to receive information and/or issue commands to the wearable medicament delivery device 1602. These devices 1630, 1633 and 1634 may execute computer programming instructions to perform some the control functions otherwise performed by processor 1614 or processor 1619. These devices 1630, 1633 and 1634 may include user interfaces, such as touchscreen displays for displaying information such as current glucose level, medicament on board such as insulin on board, medicament delivery history, or other parameters or treatment-related information and/or receiving inputs. The display may, for example, be operable to present a graphical user interface for providing input, such as request a change in basal insulin dosage or delivery of a bolus of insulin. These devices 1630, 1633 and 1634 may also have wireless communication connections with the sensor(s) 1606 to directly receive glucose level data or receive in parallel a presentation of the graphical user interface.

In another operational example, the controller 1604 may be operable to execute programming code that causes the processor 1619 of the controller 1604 to perform the following functions. The processor 1619 of the controller 1604 may execute an ADD algorithm that is one of the control applications 1620 stored in the memory 1618. The processor may be operable to present graphical user interfaces as described herein on a user interface that is at least one component of the user interface 1623. The user interface 1623 may be a touchscreen display controlled by the processor 1619, and the user interface 1623 is operable to present a graphical user interface that offers an input of a subjective insulin need parameter usable by the ADD algorithm. The processor 1619 may cause presentation on a user interface of a user device, a graphical user interface offering an input device that enables input of a subjective medicament need parameter (e.g., subjective to the user, or based on the user's desire). The ADD algorithm may generate instructions for the pump 1618 to deliver basal medicament to the user or the like.

The processor 1619 is also operable to collect physiological condition data related to the user from sensors, such as the analyte sensor(s) 1606 or heart rate data, for example, from the fitness device 1633 or the smart accessory device 1630. In an example, the processor 1619 executing the ADD algorithm may determine a dosage of medicament to be delivered based on the collected physiological condition of the user and/or a specific factor determined based on the subjective medicament need parameter. The processor 1619 may output a control signal via one of the transceivers 1625 or 1627 to the wearable medicament delivery device 1602. The outputted signal may cause the processor 1614 to deliver command signals to the pump 1618 to deliver an amount of the determined dosage of medicament in the reservoir 1620 to the user based on an output of the ADD algorithm. The processor 1619 may also be operable to perform calculations to update or establish settings of the ADD algorithm as discussed as herein. Modifications to the ADD algorithm settings may be stored in the memory 1618, for example, as settings 1621.

A wearable medicament delivery device 1602, typically, has a lifecycle that is based on the amount of liquid drug that is stored in a reservoir 1620 of the wearable medicament delivery device 1602 and/or the amount of the liquid drug delivered to the user. An ADD application or algorithm may use a number of parameters, such as glucose measurement values, total daily medicament, medicament onboard, and the like, when making the determination of an amount of the liquid drug to have delivered. In an operational example, the processor 1619 of the controller 1604 may be operable to receive evaluate the effectiveness of the ADD algorithm's control of the medicament delivery device 1602. For example, the processor 1619 may be operable to utilize historical data accessible by the processor in an evaluation of past performance of the ADD algorithm and generate recommendations for adjusting settings of the ADD algorithm accordingly as described in more detail with reference to the examples in the figures.

While the system 1600 is described with reference to delivery of insulin and the use of an ADD algorithm, the system 1600 may be operable to implement a medicament delivery regimen via a medication delivery algorithm using a number of different liquid or therapeutic drugs/medicaments. A liquid drug may be or include any drug in liquid form capable of being administered by a medicament delivery device via a subcutaneous cannula, including, for example, insulin, glucagon-like peptide-1 (GLP-1), glucose-dependent insulinotropic polypeptide (GIP), pramlintide, glucagon, co-formulations of two or more of GLP-1, GIP, pramlintide, and insulin; as well as pain relief drugs, such as opioids or narcotics (e.g., morphine, or the like), methadone, blood pressure medicines, chemotherapy drugs, fertility drugs, or the like.

In addition, or alternatively, while the examples may have been described with reference to a closed loop algorithmic implementation, variations of the disclosed examples may be implemented to enable open loop use. The open loop implementations allow for use of different modalities of delivery of insulin such as smart pen, syringe or the like. For example, the disclosed AP application and algorithms may be operable to perform various functions related to open loop operations, such as the generation of prompts requesting the input of information such as weight or age. Similarly, a dosage amount of insulin may be received by the AP application or algorithm from a user via a user interface. Other open-loop actions may also be implemented by adjusting user settings or the like in an AP application or algorithm.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of non-transitory, machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

Although the present invention is defined in the attached claims, it should be understood that the present invention can also (alternatively) be defined in accordance with the following embodiments:
1. An adhesive patch, comprising:
   a top surface configured to adhere to a wearable medical device;
   a bottom surface opposite the top surface, wherein the bottom surface includes an adhesive layer configured to adhere to a user's skin; and
   a first perforation extending through the top and bottom surfaces, the first perforation configured to receive a piercing element from the wearable medical device.
2. The adhesive patch of embodiment 1, wherein the wearable medical device includes an insulin pump.
3. The adhesive patch of embodiment 1, further comprising a second perforation configured to receive a sensing device from the wearable medical device.
4. The adhesive patch of embodiment 1, wherein the top surface includes a color corresponding to a function of the wearable medical device.
5. The adhesive patch of embodiment 1, wherein the top surface includes an adhesive layer.
6. The adhesive patch of embodiment 5, wherein the adhesive layer of the top layer is configured to adhere to an adhesive layer of a bottom of the wearable medical device.
7. The adhesive patch of embodiment 1, wherein the piercing element includes an insertion mechanism.
8. The adhesive patch of embodiment 1, wherein the adhesive layer of the bottom surface includes adhesive properties specific to a target body part of the user.
9. The adhesive patch of embodiment 8, wherein the adhesive properties include an adhesive strength specific to the target body part of the user.
10. The adhesive patch of embodiment 1, wherein the adhesive layer of the bottom surface is configured to be removed from the user's skin through a shear force applied to the adhesive layer of the bottom surface.
11. A system for a wearable medical device, comprising:
   a wearable medical device configured to deliver a medication to a user; and
   an adhesive patch coupled to the wearable medical device, comprising:
      a top surface, wherein the top surface is configured to adhere to the wearable medical device;
      a bottom surface opposite the top surface, wherein the bottom surface includes an adhesive layer configured to adhere to a user's skin; and
      a first perforation extending through the top and bottom surfaces, the first perforation configured to receive a piercing element from the wearable medical device.
12. The system of embodiment 11, wherein the wearable medical device includes an insulin pump.
13. The system of embodiment 11, further comprising a second perforation configured to receive a sensing device from the wearable medical device.
14. The system of embodiment 11, wherein the top surface includes a color corresponding to a function of the wearable medical device.
15. The system of embodiment 11, wherein the top surface includes an adhesive layer.
16. The system of embodiment 15, wherein the adhesive layer of the top layer is configured to adhere to an adhesive layer of a bottom of the wearable medical device.
17. The system of embodiment 11, wherein the piercing element includes an insertion mechanism.
18. The system of embodiment 11, wherein the adhesive layer of the bottom surface includes adhesive properties specific to a target body part of the user.
19. The system of embodiment 18, wherein the adhesive properties include an adhesive strength specific to the target body part of the user.
20. The system of embodiment 11, wherein the adhesive layer of the bottom surface is configured to be removed from the user's skin through a shear force applied to the adhesive layer of the bottom surface.
21. A kit of parts comprising:
   a first adhesive patch and a second adhesive patch, wherein the first adhesive patch and the second adhesive patch each comprise:
   a top surface configured to adhere to a wearable medical device;
   a bottom surface opposite the top surface, wherein the bottom surface includes an adhesive layer configured to adhere to a user's skin; and
   a first perforation extending through the top and bottom surfaces, the first perforation configured to receive a piercing element from the wearable medical device;
   and wherein the first adhesive patch and the second adhesive patch differ by at least one of and adhesive strength, a colour, dimensions or perforation position.
22. The kit of parts according to embodiment 21, wherein the kit of parts comprises a wearable medical device, more specifically a disposable wearable medical device, and in particular a disposable wearable insulin delivery device.
23. The kit of parts according to any one of embodiments 21 to 22, wherein the first adhesive patch has a different adhesive strength and a different color than the second adhesive patch.
24. The kit of parts according to any one of embodiments 21 to 23, wherein the first adhesive patch and the second adhesive patch comprise a release liner attached to the adhesive layer.
25. A kit of parts comprising:
   a wearable drug delivery device comprising a reservoir in fluid communication with a port, and a reservoir filling kit,
   wherein the reservoir filling kit comprises a filling kit marker,
   and wherein the port comprises a port marker, wherein the filling kit marker and the port marker correspond to one another.
26. The kit of parts according to embodiment 24, wherein the reservoir filling kit comprises a vial and a syringe, wherein the vial comprises the filling kit marker, and
   optionally, wherein the syringe comprises a syringe marker that corresponds to the port marker and filling marker.
27. The kit of parts according to embodiment 26, wherein the port marker and the filling kit marker are colored markers, wherein the port marker and the filling kit marker have the same color, and
   optionally, wherein the syringe marker is also a color marker with the same color as the filling kit marker.
28. The kit of parts according to embodiment 25 to 27, wherein the port marker is arranged around or next to an opening of the port.
29. A wearable drug delivery device, wherein the wearable drug delivery device comprises:
   a first reservoir in fluid communication with a first opening of a first reservoir port comprising a first port marker,
   a second reservoir in fluid communication with a second opening of a second reservoir port comprising a second port marker, wherein the first port marker and the second port marker are different.
30. A kit of parts comprising the wearable drug delivery device of embodiment 30,
   a first filling kit comprising a first filling kit marker corresponding to the first port marker, and
   a second filling kit comprising a second filling kit marker corresponding to the second port marker.
31. The kit of parts according to embodiment 30, wherein the first filling kit comprises a first vial comprising the first filling kit color marker and wherein the second filling kit comprises a second vial comprising the second filling kit color marker, and wherein the kit of parts additionally comprises a syringe comprising two graduations, wherein the first graduation comprises graduation markers in the color of the first port color marker and the second graduation comprises graduation markers in the color of the second port color marker.
32. A wearable drug delivery device, wherein the wearable drug delivery device comprises:
   a first reservoir in fluid communication with a first reservoir port, wherein the first reservoir comprises a first viewing window, wherein a first viewing window label is arranged around or next to the first viewing window; and, optionally,
   a second reservoir in fluid communication with a second reservoir port, wherein the second reservoir comprises a second viewing window, wherein a second viewing window label is arranged around or next to the first viewing window; and wherein the first viewing window label and the second viewing window label are different, in particular wherein the first viewing window label and the second viewing window label have different colors.
33. A kit of parts comprising the drug delivery device according to embodiment 32, wherein the kit of parts additionally comprises a first vial comprising a first fluid drug and a second vial comprising a second fluid drug,
   wherein the first vial has a first vial marker corresponding to the first viewing window label, and wherein the second vial marker has a second vial marker corresponding to the second viewing window label; and/or,
   wherein the first fluid drug has a color corresponding to the color of the first viewing window label and wherein the second fluid drug has a color corresponding to the second viewing window label.
34. A wearable drug delivery device, wherein the wearable drug delivery device comprises:
   a first reservoir in fluid communication with a first reservoir port, wherein the first reservoir port comprises a first port adapter; and, optionally,
   a second reservoir in fluid communication with a second reservoir port, wherein the second reservoir port comprises a second port adapter.
35. A kit of parts comprising a drug delivery device according to embodiment 34, wherein the kit of parts additionally comprises a first syringe comprising a first syringe key adapter configured to fit with the first port adapter, such that the first syringe can be introduced into the first port and wherein the first syringe key adapter is additionally configured to not fit with the second port adapter such that the first syringe cannot be introduced in the second port; and wherein the kit of parts additionally comprises a second syringe comprising a second syringe key adapter configured to fit with the second port adapter, such that the second syringe can be introduced into the second port and wherein the second syringe adapter is additionally configured to not fit with the first port adapter such that the second syringe cannot be introduced in the first port.

## Claims

1. An adhesive patch, comprising:
a top surface configured to adhere to a wearable medical device;
a bottom surface opposite the top surface, wherein the bottom surface includes an adhesive layer configured to adhere to a user's skin; and
a first perforation extending through the top and bottom surfaces, the first perforation configured to receive a piercing element from the wearable medical device.

2. The adhesive patch of claim 1, wherein the wearable medical device includes an insulin pump.

3. The adhesive patch of claim 1, further comprising a second perforation configured to receive a sensing device from the wearable medical device.

4. The adhesive patch of claim 1, wherein the top surface includes a color corresponding to a function of the wearable medical device.

5. The adhesive patch of claim 1, wherein the top surface includes an adhesive layer.

6. The adhesive patch of claim 5, wherein the adhesive layer of the top layer is configured to adhere to an adhesive layer of a bottom of the wearable medical device.

7. The adhesive patch of claim 1, wherein the piercing element includes an insertion mechanism.

8. The adhesive patch of claim 1, wherein the adhesive layer of the bottom surface includes adhesive properties specific to a target body part of the user.

9. The adhesive patch of claim 8, wherein the adhesive properties include an adhesive strength specific to the target body part of the user.

10. The adhesive patch of claim 1, wherein the adhesive layer of the bottom surface is configured to be removed from the user's skin through a shear force applied to the adhesive layer of the bottom surface.

11. A system for a wearable medical device, comprising:
a wearable medical device configured to deliver a medication to a user; and
an adhesive patch coupled to the wearable medical device, comprising:
a top surface, wherein the top surface is configured to adhere to the wearable medical device;
a bottom surface opposite the top surface, wherein the bottom surface includes an adhesive layer configured to adhere to a user's skin; and
a first perforation extending through the top and bottom surfaces, the first perforation configured to receive a piercing element from the wearable medical device.

12. The system of claim 11, further comprising a second perforation configured to receive a sensing device from the wearable medical device.

13. The system of claim 11, wherein the top surface includes a color corresponding to a function of the wearable medical device.

14. The system of claim 11, wherein the adhesive layer of the bottom surface includes adhesive properties specific to a target body part of the user.

15. The system of claim 11, wherein the adhesive layer of the bottom surface is configured to be removed from the user's skin through a shear force applied to the adhesive layer of the bottom surface.
